(19) Europäisches Patentamt
European Patent Office
Office européen des brevets

(11) EP 2 900 689 B1

(12) **EUROPEAN PATENT SPECIFICATION**

(45) Date of publication and mention of the grant of the patent:
**02.08.2017 Bulletin 2017/31**

(21) Application number: **13773567.6**

(22) Date of filing: **26.09.2013**

(51) Int Cl.:
*C07K 14/32* (2006.01) *C12P 21/00* (2006.01)
*C12P 21/02* (2006.01) *C12N 15/75* (2006.01)
*C12P 5/00* (2006.01) *C12P 5/02* (2006.01)
*C12P 7/04* (2006.01) *C12P 13/04* (2006.01)

(86) International application number:
**PCT/US2013/061980**

(87) International publication number:
**WO 2014/052630 (03.04.2014 Gazette 2014/14)**

(54) **BACTERIAL MUTANTS WITH IMPROVED TRANSFORMATION EFFICIENCY**

BAKTERIELLE MUTANTEN MIT VERBESSERTER TRANSFORMATIONSEFFIZIENZ

MUTANTS BACTÉRIENS DOTÉS D'UNE EFFICACITÉ DE TRANSFORMATION AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2012 US 201261706661 P**

(43) Date of publication of application:
**05.08.2015 Bulletin 2015/32**

(73) Proprietor: **Novozymes, Inc.**
**Davis, CA 95618 (US)**

(72) Inventors:
- **BERKA, Randy**
**Davis, California 95618 (US)**
- **CHERRY, Barbara**
**Winters, California 95694 (US)**

(74) Representative: **Lindum, Peter Würtz**
**Novozymes A/S**
**Patents**
**Krogshøjvej 36**
**2880 Bagsværd (DK)**

(56) References cited:
**WO-A2-2008/079895**

- **J. HAHN ET AL: "Inactivation of mecA prevents recovery from the competent state and interferes with cell division and the partitioning of nucleoids in Bacillus subtilis", MOLECULAR MICROBIOLOGY, vol. 18, no. 4, 15 November 1995 (1995-11-15), pages 755-767, XP055088876, ISSN: 0950-382X, DOI: 10.1111/j.1365-2958.1995.mmi_18040755.x cited in the application**
- **ASHIKAGA SAYAKA ET AL: "Natural genetic competence in Bacillus subtilis natto OK2", JOURNAL OF BACTERIOLOGY, AMERICAN SOCIETY FOR MICROBIOLOGY, WASHINGTON, DC; US, vol. 182, no. 9, May 2000 (2000-05), pages 2411-2415, XP002488123, ISSN: 0021-9193, DOI: 10.1128/JB.182.9.2411-2415.2000**
- **L. W. HAMOEN: "Controlling competence in Bacillus subtilis: shared use of regulators", MICROBIOLOGY, vol. 149, no. 1, January 2003 (2003-01), pages 9-17, XP055088717, ISSN: 1350-0872, DOI: 10.1099/mic.0.26003-0**
- **NICOLAS MIROUZE ET AL: "Spo0A~ P Imposes a Temporal Gate for the Bimodal Expression of Competence in Bacillus subtilis", PLOS GENETICS, vol. 8, no. 3, 8 March 2012 (2012-03-08), page e1002586, XP055088807, DOI: 10.1371/journal.pgen.1002586**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- PETER PREPIAK ET AL: "MecA dampens transitions to spore, biofilm exopolysaccharide and competence expression by two different mechanisms", MOLECULAR MICROBIOLOGY, vol. 80, no. 4, 11 April 2011 (2011-04-11) , pages 1014-1030, XP055088802, ISSN: 0950-382X, DOI: 10.1111/j.1365-2958.2011.07627.x cited in the application
- TRAN THU HOA ET AL: "Rok (YkuW) regulates genetic competence in Bacillus subtilis by directly repressing comK", MOLECULAR MICROBIOLOGY, vol. 43, no. 1, January 2002 (2002-01), pages 15-26, XP055088796, ISSN: 0950-382X, DOI: 10.1046/j.1365-2958.2002.02727.x
- U BAI ET AL: "SinI modulates the activity of SinR, a developmental switch protein of Bacillus subtilis, by protein-protein interaction.", GENES & DEVELOPMENT, vol. 7, no. 1, January 1993 (1993-01), pages 139-148, XP055088801, ISSN: 0890-9369, DOI: 10.1101/gad.7.1.139

## Description

### Reference to a Sequence Listing

This application contains a Sequence Listing in computer readable form, which is incorporated herein by reference.

### Background

Genetic competence is a physiological state in which exogenous DNA can be internalized, leading to a transformation event (Berka et al., Mol. Microbiol. 2002, 43,1331-1345), but is distinct from artificial transformation involving electroporation, protoplasts, and heat shock or $CaCl_2$ treatment. Natural competence has been observed in both Gram positive and Gram negative bacterial species (Dubnau, Annual Rev. Microbiol. 1999, 53, 217-244), and the process requires more than a dozen proteins whose expression is precisely choreographed to the needs of each organism.

Several hypotheses have been proposed regarding the purpose of natural competence, and they can be summarized as DNA for food, DNA for repair, and DNA for genetic diversity (Dubnau, 1999, *supra).* The DNA for food hypothesis is supported by observations that competence is a stationary phase phenomenon that occurs when cells are nutrient limited, and often a powerful nonspecific nuclease is co-expressed with transformation specific proteins. Evidence for the second hypothesis comes from the fact that genes encoding DNA repair enzymes are coordinately expressed with those encoding DNA transport proteins. Lastly, the DNA for genetic diversity hypothesis proposes that competence is a mechanism for exploring the fitness landscape via horizontal gene transfer. Observations that competence is regulated by a quorum-sensing mechanism and that it is a bistable condition (Avery, Trends Microbiol. 2005, 13, 459-462) support this hypothesis.

Public databases now contain a multitude of complete bacterial genomes, including several genomes from different strains of the same species. Recent analyses have shown, using pairwise whole genome alignments, that different strains of the same species may vary substantially in gene content. For example, genome comparisons of *Escherichia coli* strains CFT073, EDL933, and MG1655 revealed that only 39.2% of their combined set of proteins (gene products) are common to all three strains, highlighting the astonishing diversity among strains of the same species (Blattner et al., Science 1997, 277, 1453-1474; Hayashi et al., 2006, Mol. Syst. Biol. doi:10.1038:msb4100049; Perna et al., Nature 2001, 409, 529-533; Welch et al., Proc. Natl. Acad Sci. USA 2002, 99, 17020-17024). Furthermore, the genome sequence of *E. coli* strain CFT073 revealed 1,623 strain-specific genes (21.2%). From comparisons of this type, it is clearly seen that bacterial genomes are segmented into a common conserved backbone and strain-specific sequences. Typically the genome of a given strain within a species shows a mosaic structure in terms of the distribution of conserved "backbone" genes conserved among all strains and non-conserved genes that may have been acquired by horizontal transfer (Brzuszkiewicz et al., Proc. Natl. Acad. Sci. USA 2006, 103, 12879-12884; Welch et al., 2002, *supra*).

In terms of practical utility, transformation via natural competence is an extremely useful tool for constructing bacterial strains, e.g., *Bacillus,* that may contain altered alleles for chromosomal genes or plasmids assembled via recombinant DNA methods. Although transformation of certain species with plasmids and chromosomal DNA may be achieved via artificial means as noted above (e.g., electroporation, protoplasts, and heat shock or $CaCl_2$ treatment), introduction of DNA by natural competence offers clear advantages of simplicity, convenience, speed, and efficiency.

In *Bacillus subtilis,* only 5-10% of the cells in a population differentiate to a competent state (termed the *K-state*) via a process that involves quorum-sensing, signal transduction, and a cascade of gene expression (Avery, 2005, *supra*). At least 50 genes are known to be involved directly in competence, and as many as 165 genes are regulated (directly or indirectly) by the central transcription factor ComK (Berka et al., 2002, *supra*). The competence cascade in *Bacillus subtilis* consists of two regulatory modules punctuated by a molecular switch (Figure 1) that involves ComS binding to the adaptor molecule MecA, thereby interfering with degradation of the transcription factor ComK by the ClpC/ClpP protease (Turgay et al., EMBO J. 1998, 17, 6730-6738).

In *Bacillus subtilis, mecA* inactivation has been shown to moderately elevate transformation efficiency due to increased availability of ComK (Hahn et. al, Mol. Microbiol., 1995, 18, 755-767). A recent report suggests a *Bacillus subtilis mecA* deletion results in an increased expression of the *eps* and *tasA* operons (Prepiak et. al, Mol. Microbiol., 2011, 80, 1014-1030) in accordance with the regulatory relationship between *mecA* and the *eps* and *tasA* regulons shown in Figure 2.

With the exception of *comP* and *comS*, *Bacillus licheniformis* harbors orthologues of the genes necessary to achieve natural competence. Ostensibly naturally competent *Bacillus licheniformis* cells cannot be obtained due to the lack of a functional *comS* gene resulting in ComK continually sequestered by MecA, and proteolytically degraded by ClpC/P/MecA complex. Applicants have shown that expression of ComS and ComK in *Bacillus licheniformis* can improve competence (US2010/0028944).

Since *Bacillus* species provide a key platform for a variety of industrially relevant processes, such as metabolic engineering and biochemical production, engineering strains that manifest improved competence is highly desirable for construction of new and improved production strains. The availability of a turn-key method for improving competence in *Bacillus* strains would improve the speed and efficiency with which chromosomal markers/alleles and expression vectors could be introduced. The present invention fulfills these and other needs.

## Summary

Described herein are *Bacillus* mutants having improved transformation efficiency. The Applicants have surprisingly found that disruption to both endogenous genes *mecA* and *sinI* in a *Bacillus* host cell shows significantly improved transformation efficiency compared to disruption of the endogenous *mecA* gene alone.

In one aspect is a mutant of a parent *Bacillus* strain, comprising disruption to an endogenous *mecA* gene native to the parent *Bacillus* strain and disruption to an endogenous *sinI* gene native to the parent *Bacillus* strain, wherein the endogenous *mecA* gene native to the parent *Bacillus* strain encodes for a polypeptide having at least 80% sequence identity to SEQ ID NO: 2 or 16, wherein the endogenous *sinI* gene native to the parent *Bacillus* strain encodes for a polypeptide having at least 80% sequence identity to SEQ ID NO: 4 or 18, and wherein the mutant is capable of producing at least 10-fold more transformants compared to the parent *Bacillus* strain that lacks disruption to the endogenous *mecA* gene and lacks disruption to the endogenous *sinI* gene, when cultivated under identical conditions. In some aspects, the *Bacillus* mutant is a *Bacillus licheniformis* mutant or a *Bacillus subtilis* mutant.

Also described are methods of producing a polypeptide of interest, comprising: (a) cultivating a *Bacillus* mutant of the first aspect comprising a heterologous polynucleotide encoding the polypeptide of interest; and (b) recovering the polypeptide.

## Brief Description of the Figures

Figure 1 shows the competence regulatory cascade of *Bacillus subtilis.* Module 1 involves detection of the competence pheromone CSF and signal transduction via a phosphorelay mechanism resulting in synthesis of the ComS peptide. ComS interferes with proteolytic degradation of the transcription factor ComK via binding to MecA that activates Module 2 encoding the late competence functions encoding DNA transport machinery.

Figure 2 shows the regulatory relationship between *mecA* and the *eps* and *tasA* regulons in *Bacillus subtilis.*

Figure 3 shows the DNA sequence and the deduced amino acid sequence of the *Bacillus licheniformis mecA* gene (SEQ ID NOs: 1 and 2, respectively).

Figure 4 shows the DNA sequence and the deduced amino acid sequence of the *Bacillus licheniformis sinI* gene (SEQ ID NOs: 3 and 4, respectively).

Figure 5 shows the DNA sequence and the deduced amino acid sequence of the *Bacillus subtilis mecA* gene (SEQ ID NOs: 15 and 16, respectively).

Figure 6 shows the DNA sequence and the deduced amino acid sequence of the *Bacillus subtilis sinI* gene (SEQ ID NOs: 17 and 18, respectively).

Figure 7 shows the transformation efficiency of strains TaHy9 comprising a *mecA* gene disruption.

Figure 8 shows the relative transformation efficiency of strains TaHy9 (comprising a *mecA* gene disruption) and BaC0155 (comprising a *mecA* gene disruption and a *sinI* gene disruption).

## Definitions

**Disruption:** The term "disruption" means that a coding region and/or control sequence of a referenced gene is partially or entirely modified (such as by deletion, insertion, and/or substitution of one or more nucleotides, or by association with RNAi or antisense technology) resulting in the absence (inactivation) or decrease in expression, and/or the absence or decrease of enzyme activity of the encoded polypeptide. The effects of disruption can be measured using techniques known in the art such as detecting the absence or decrease of enzyme activity using from cell-free extract measurements referenced herein; or by the absence or decrease of corresponding mRNA (e.g., at least 25% decrease, at least 50% decrease, at least 60% decrease, at least 70% decrease, at least 80% decrease, or at least 90% decrease); the absence or decrease in the amount of corresponding polypeptide having enzyme activity (e.g., at least 25% decrease, at least 50% decrease, at least 60% decrease, at least 70% decrease, at least 80% decrease, or at least 90% decrease); or the absence or decrease of the specific activity of the corresponding polypeptide having enzyme activity (e.g., at least 25% decrease, at least 50% decrease, at least 60% decrease, at least 70% decrease, at least 80% decrease, or at least 90% decrease). Disruptions of a particular gene of interest can be generated by methods known in the art, e.g., by directed homologous recombination (see Methods in Yeast Genetics (1997 edition), Adams, Gottschling, Kaiser, and Stems, Cold Spring Harbor Press (1998)). Techniques to disrupt *Bacillus* genes are described herein and have been demonstrated in the art (see Stahl & Ferrari, J. Bacteriol. 1984, 158,411-418).

**Parent:** The term "parent" or "parent *Bacillus* strain" means a *Bacillus* strain to which a disruption is made to produce a mutant *Bacillus* strain described herein. The parent may be a naturally occurring (wild-type) or previously modified *Bacillus* strain.

**Mutant:** The term "mutant" means the resulting *Bacillus* strain after one or more disruptions are made to a parent *Bacillus* strain.

**Improved transformation efficiency:** The term "improved transformation efficiency" means that the referenced *Bacillus* mutant strain is capable of generating an increased number of transformants compared to the parent *Bacillus* strain when transformed and cultivated under identical conditions. Improved transformation efficiency can be demonstrated by generating an increased number of transformants using transformation methods described in the Examples below. Improved transformation efficiency may also be demonstrated using methods previously described (e.g., Anagnostopoulos and Spizizen, J. Bacteriol. 1961, 81, 741-746). In some aspects, the *Bacillus* mutant strain is capable of producing at least 20-fold, at least 50-fold, at least 100-fold, at least 200-fold, at least 500-fold, at least 1000-fold, at least 2000-fold, at least 5000-fold, at least 10000-fold, at least 20000-fold, at least 50000-fold, or at least 100000-fold more transformants compared to the parent *Bacillus* strain that lacks disruption to the endogenous *mecA* gene and lacks disruption to the endogenous *sinl* gene, when cultivated under identical conditions.

**Coding sequence:** The term "coding sequence" means a polynucleotide sequence, which specifies the amino acid sequence of a polypeptide. The boundaries of the coding sequence are generally determined by an open reading frame, which usually begins with the ATG start codon or alternative start codons such as GTG and TTG and ends with a stop codon such as TAA, TAG, and TGA. The coding sequence may be a sequence of genomic DNA, cDNA, a synthetic polynucleotide, and/or a recombinant polynucleotide.

**Sequence Identity:** The relatedness between two amino acid sequences or between two nucleotide sequences is described by the parameter "sequence identity".

For purposes described herein, the degree of sequence identity between two amino acid sequences is determined using the Needleman-Wunsch algorithm (Needleman and Wunsch, J. Mol. Biol. 1970, 48, 443-453) as implemented in the Needle program of the EMBOSS package (EMBOSS: The European Molecular Biology Open Software Suite, Rice et al., Trends Genet 2000, 16, 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes described herein, the degree of sequence identity between two deoxyribonucleotide sequences is determined using the Needleman-Wunsch algorithm (Needleman and Wunsch, **1970**, *supra*) as implemented in the Needle program of the EMBOSS package (EMBOSS: The European Molecular Biology Open Software Suite, Rice et al., 2000, supra), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides} \times 100)/(\text{Length of Alignment} - \text{Total Number of Gaps in Alignment})$$

**Hybridization conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 0.2X SSC, 0.2% SDS at 45°C.

The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 0.2X SSC, 0.2% SDS at 50°C.

The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 0.2X SSC, 0.2% SDS at 55°C.

The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 0.2X SSC, 0.2% SDS at 60°C.

The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 0.2X SSC, 0.2% SDS at 65°C.

The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 0.2X SSC, 0.2% SDS at 70°C.

**Heterologous polynucleotide:** The term "heterologous polynucleotide" is defined herein as a polynucleotide that is not native to the host cell; a native polynucleotide in which one or more (e.g., two, several) structural modifications have been made to the coding region; a native polynucleotide whose expression is quantitatively altered as a result of manipulation of the DNA by recombinant DNA techniques, e.g., a different (foreign) promoter linked to the polynucleotide; or a native polynucleotide whose expression is quantitatively altered by the introduction of one or more extra copies of the polynucleotide into the host cell.

**Isolated:** The term "isolated" means a substance in a form or environment that does not occur in nature. Non-limiting examples of isolated substances include (1) any non-naturally occurring substance, (2) any substance including, but not limited to, any host cell, enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated.

**Endogenous gene:** The term "endogenous gene" means a gene that is native to the parent *Bacillus* strain.

**Nucleic acid construct:** The term "nucleic acid construct" means a polynucleotide comprises one or more (e.g., two, several) control sequences. The polynucleotide may be single-stranded or double-stranded, and may be isolated from a naturally occurring gene, modified to contain segments of nucleic acids in a manner that would not otherwise exist in nature, or synthetic.

**Control sequence:** The term "control sequence" means a nucleic acid sequence necessary for polypeptide expression. Control sequences may be native or foreign to the polynucleotide encoding the polypeptide, and native or foreign to each other. Such control sequences include, but are not limited to, a leader sequence, polyadenylation sequence, propeptide sequence, promoter sequence, signal peptide sequence, and transcription terminator sequence. The control sequences may be provided with linkers for the purpose of introducing specific restriction sites facilitating ligation of the control sequences with the coding region of the polynucleotide encoding a polypeptide.

**Operably linked:** The term "operably linked" means a configuration in which a control sequence is placed at an appropriate position relative to the coding sequence of a polynucleotide such that the control sequence directs the expression of the coding sequence.

**Expression:** The term "expression" includes any step involved in the production of the polypeptide including, but not limited to, transcription, post-transcriptional modification, translation, post-translational modification, and secretion. Expression can be measured-for example, to detect increased expression-by techniques known in the art, such as measuring levels of mRNA and/or translated polypeptide.

**Expression vector:** The term "expression vector" means a linear or circular DNA molecule that comprises a polynucleotide encoding a polypeptide and is operably linked to control sequences, wherein the control sequences provide for expression of the polynucleotide encoding the polypeptide. At a minimum, the expression vector comprises a promoter sequence, and transcriptional and translational stop signal sequences.

**Host cell:** The term "host cell" means any cell type that is susceptible to transformation, transfection, transduction, and the like with a nucleic acid construct or expression vector. The term "host cell" encompasses any progeny of a parent cell that is not identical to the parent cell due to mutations that occur during replication.

**Transformation:** The term "transformation" means introducing a heterologous polynucleotide into a *Bacillus* cell so that the DNA is maintained as a chromosomal integrant or as a self-replicating extra-chromosomal vector. The resulting *Bacillus* cell following transformation is described herein as a "transformant."

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

Reference to "about" a value or parameter herein includes aspects that are directed to that value or parameter *per se.* For example, description referring to "about X" includes the aspect "X". When used in combination with measured values, "about" includes a range that encompasses at least the uncertainty associated with the method of measuring the particular value, and can include a range of plus or minus two standard deviations around the stated value.

As used herein and in the appended claims, the singular forms "a," "or," and "the" include plural referents unless the context clearly dictates otherwise. It is understood that the aspects described herein include "consisting" and/or "consisting essentially of" aspects.

Unless defined otherwise or clearly indicated by context, all technical and scientific terms used herein have the same meaning as commonly understood by one of ordinary skill in the art.

## Detailed Description

### *Bacillus* Mutants

Described herein, *inter alia*, are mutants of a parent *Bacillus* strain, comprising a disruption to an endogenous *mecA* gene native to the parent *Bacillus* strain and disruption to an endogenous *sinl* gene native to the parent *Bacillus* strain, wherein the mutant has improved transformation efficiency.

The parent strain of the mutants and related methods may be any *Bacillus* strain, such as a wild-type *Bacillus* or a mutant thereof. In some aspects, the parent *Bacillus* strain is a *Bacillus alkalophilus*, *Bacillus amyloliquefaciens*, *Bacillus brevis*, *Bacillus circulans*, *Bacillus clausii*, *Bacillus coagulans*, *Bacillus firmus*, *Bacillus lautus*, *Bacillus lentus*, *Bacillus licheniformis*, *Bacillus megaterium*, *Bacillus pumilus*, *Bacillus stearothermophilus*, *Bacillus subtilis*, or *Bacillus thuringiensis* strain.

The disrupted gene may be any suitable endogenous *Bacillus mecA* gene native to the parent *Bacillus* strain and *sinl* gene native to the parent *Bacillus* strain. Examples of target genes native to the parent *Bacillus* strain include the *Bacillus licheniformis mecA* gene of SEQ ID NO: 1 (encoding the polypeptide of SEQ ID NO: 2), the *Bacillus licheniformis sinl* gene of SEQ ID NO: 3 (encoding the polypeptide of SEQ ID NO: 4), *Bacillus subtilis mecA* gene of SEQ ID NO: 15 (encoding the polypeptide of SEQ ID NO: 16), and the *Bacillus subtilis sinl* gene of SEQ ID NO: 17 (encoding the polypeptide of SEQ ID NO: 18).

In some aspects of the mutants and related methods, the endogenous *mecA* gene native to the parent *Bacillus* strain encodes for a polypeptide having at least 80%, at least 85%, at least 90%, at least 95%, at least 97%, at least 98%, at least 99%, or 100% sequence identity to SEQ ID NO: 2 or 16. In some aspects, the endogenous *mecA* gene native to the parent *Bacillus* strain encodes for a polypeptide having a sequence that differs by no more than ten amino acids, e.g., by no more than five amino acids, by no more than four amino acids, by no more than three amino acids, by no more than two amino acids, or by one amino acid from SEQ ID NO: 2 or 16. In some aspects, the endogenous *mecA* gene native to the parent *Bacillus* strain encodes for a polypeptide comprising or consisting of SEQ ID NO: 2 or 16.

In other aspects of the mutants or related methods, the endogenous *mecA* gene native to the parent *Bacillus* strain comprises a coding sequence that has at least 80%, at least 85%, at least 90%, at least 95%, at least 97%, at least 98%, at least 99%, or 100% sequence identity to SEQ ID NO: 1 or 15. In some aspects, the coding sequence of the endogenous *mecA* gene native to the parent *Bacillus* strain comprises or consists of SEQ ID NO: 1 or 15.

In other aspects of the mutants or related methods, the endogenous *mecA* gene native to the parent *Bacillus* strain comprises a coding sequence that hybridizes under at least medium stringency conditions, medium-high stringency conditions, high stringency conditions, or very high stringency conditions with the full-length complementary strand of SEO ID NO: 1 or 15.

In some aspects of the mutants and related methods, the endogenous *sinl* gene native to the parent *Bacillus* strain encodes for a polypeptide having at least 80%, at least 85%, at least 90%, at least 95%, at least 97%, at least 98%, at least 99%, or 100% sequence identity to SEQ ID NO: 4 or 18. In some aspects, the endogenous *sinl* gene native to the parent *Bacillus* strain encodes for a polypeptide having a sequence that differs by no more than ten amino acids, e.g., by no more than five amino acids, by no more than four amino acids, by no more than three amino acids, by no more than two amino acids, or by one amino acid from SEQ ID NO: 4 or 18. In some aspects, the endogenous *sinl* gene native to the parent *Bacillus* strain encodes for a polypeptide comprising or consisting of SEQ ID NO: 4 or 18.

In other aspects of the mutants or related methods, the endogenous *sinl* gene native to the parent *Bacillus* strain comprises a coding sequence that has at least 80%, at least 85%, at least 90%, at least 95%, at least 97%, at least 98%, at least 99%, or 100% sequence identity to SEQ ID NO: 3 or 17. In some aspects, the coding sequence of the endogenous *sinl* gene native to the parent *Bacillus* strain comprises or consists of SEQ ID NO: 3 or 17.

In other aspects of the mutants or related methods, the endogenous *sinl* gene native to the parent *Bacillus* strain comprises a coding sequence that hybridizes under at least medium stringency conditions, medium-high stringency conditions, high stringency conditions, or very high stringency conditions with the full-length complementary strand of SEQ ID NO: 3 or 17.

The polynucleotide sequences disclosed herein, or a subsequences thereof; as well as the amino acid sequences described herein, of or a fragment thereof; may be used to design nucleic acid probes to identify and clone homologous *mecA* and *sinI* genes from strains of different genera or species according to methods well known in the art. In particular, such probes can be used for hybridization with the DNA from a *Bacillus* species of interest, following standard Southern blotting procedures, in order to identify and isolate the corresponding gene therein. Such probes can be considerably shorter than the entire sequence, e.g., at least 14 nucleotides, at least 25 nucleotides, at least 35 nucleotides, at least 70 nucleotides in lengths. The probes may be longer, e.g., at least 100 nucleotides, at least 200 nucleotides, at least 300 nucleotides, at least 400 nucleotides, at least 500 nucleotides in lengths. Even longer probes may be used, e.g., at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin).

A DNA library prepared from such other strains may be screened for DNA that hybridizes with the probes described above. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that is homologous with the polynucleotide sequences described herein, or a subsequence thereof, the carrier material may be used in a Southern blot. For purposes of the probes described above, hybridization indicates that the polynucleotide hybridizes to a labeled nucleic acid probe corresponding to the polynucleotide sequences, the full-length complementary strand thereof, or a subsequence of the foregoing; under very low to very high stringency conditions. Molecules to which the nucleic acid probe hybridizes under these conditions can be detected using, for example, X-ray film.

For long probes of at least 100 nucleotides in length, very low to very high stringency conditions are defined as prehybridization and hybridization at 42°C in 5X SSPE, 0.3% SDS, 200 micrograms/mL sheared and denatured salmon sperm DNA, and either 25% formamide for very low and low stringencies, 35% formamide for medium and medium-high stringencies, or 50% formamide for high and very high stringencies, following standard Southern blotting procedures for 12 to 24 hours optimally. The carrier material is finally washed three times each for 15 minutes using 2X SSC, 0.2% SDS at 45°C (very low stringency), at 50°C (low stringency), at 55°C (medium stringency), at 60°C (medium-high stringency), at 65°C (high stringency), and at 70°C (very high stringency).

For short probes of about 15 nucleotides to about 70 nucleotides in length, stringency conditions are defined as prehybridization and hybridization at about 5°C to about 10°C below the calculated $T_m$ using the calculation according to Bolton and McCarthy (Proc. Natl. Acad. Sci. USA 1962, 48, 1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per mL following standard Southern blotting procedures for 12 to 24 hours optimally. The carrier material is finally washed once in 6X SCC plus 0.1% SDS for 15 minutes and twice each for 15 minutes using 6X SSC at 5°C to 10°C below the calculated $T_m$.

Homologs of the polypeptides encoded by the endogenous *mecA* and *sinI* genes described herein from strains of different genera or species generally have amino acid changes that are of a minor nature, that is conservative amino acid substitutions or insertions that do not significantly affect the folding and/or activity of the protein; small deletions, typically of one to about 30 amino acids; small amino-terminal or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids can be identified according to procedures known in the art, such as site-directed mutagenesis or alanine-scanning mutagenesis (Cunningham and Wells, Science 1989, 244, 1081-1085). In the latter technique, single alanine mutations are introduced at every residue in the molecule, and the resultant mutant molecules are tested for activity to identify amino acid residues that are critical to the activity of the molecule. See also, Hilton et al., J. Biol. Chem. 1996, 271, 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., Science 1992, 255, 306-312; Smith et al., J. Mol. Biol. 1992, 224, 899-904; Wlodaver et al., FEBS Lett. 1992, 309, 59-64. The identities of essential amino acids can also be inferred from analysis of identities with other related enzymes.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, Science 1988, 241, 53-57; Bowie and Sauer, Proc. Natl. Acad. Sci. U.S.A. 1989, 86, 2152-2156; WO 95/17413; or WO 95/22625. Other methods that can be used include error-prone PCR, phage display (e.g., Lowman et al., Biochemistry 1991, 30, 10832-10837; U.S. Patent No. 5,223,409; WO 92/06204), and region-directed mutagenesis (Derbyshire et al., Gene 1986, 46, 145; Ner et al., DNA 1988, 7, 127).

Mutagenesis/shuffling methods can be combined with high-throughput, automated screening methods to detect activity of cloned, mutagenized polypeptides expressed by host cells (Ness et al., Nature Biotechnol. 1999, 17, 893-896). Mutagenized DNA molecules that encode active enzymes can be recovered from the host cells and rapidly sequenced using standard methods in the art. These methods allow the rapid determination of the importance of individual amino acid residues in a polypeptide.

**Disruption of *mecA* and *sinI* Genes and Methods of Producing *Bacillus* Mutants**

The *Bacillus* mutant strains described herein may be constructed by disrupting the referenced endogenous *mecA* and *sinI* genes native to the parent *Bacillus* strain using methods well known in the art, including those methods described herein. A portion of the gene can be disrupted such as the coding region or a control sequence required for expression of the coding region. Such a control sequence of the gene may be a promoter sequence or a functional part thereof, i.e., a part that is sufficient for affecting expression of the gene. For example, a promoter sequence may be inactivated resulting in no expression or a weaker promoter may be substituted for the native promoter sequence to reduce expression of the coding sequence. Other control sequences for possible modification include, but are not limited to, a leader, propeptide sequence, signal sequence, transcription terminator, and transcriptional activator.

The *Bacillus* mutant strains may be constructed by gene deletion techniques to eliminate or reduce expression of the gene. Gene deletion techniques enable the partial or complete removal of the gene thereby eliminating expression. In such methods, deletion of the gene is accomplished by homologous recombination using a plasmid that has been constructed to contiguously contain the 5' and 3' regions flanking the gene.

The *Bacillus* mutant strains may also be constructed by introducing, substituting, and/or removing one or more (e.g., two, several) nucleotides in the gene or a control sequence thereof required for the transcription or translation thereof. For example, nucleotides may be inserted or removed for the introduction of a stop codon, the removal of the start codon, or a frame-shift of the open reading frame. Such a modification may be accomplished by site-directed mutagenesis or PCR generated mutagenesis in accordance with methods known in the art. See, for example, Botstein and Shortle, Science 1985, 229, 4719; Lo et al., Proc. Natl. Acad. Sci. U.S.A. 1985, 81, 2285; Higuchi et al., Nucleic Acids Res 1988, 16, 7351; Shimada, Meth. Mol. Biol. 1996, 57, 157; Ho et al., Gene 1989, 77, 61; Horton et al., Gene 1989, 77, 61; and Sarkar and Sommer, BioTechniques 1990, 8, 404.

The *Bacillus* mutant strains may also be constructed by gene disruption techniques by inserting into the gene a disruptive nucleic acid construct comprising a nucleic acid fragment homologous to the gene that will create a duplication of the region of homology and incorporate construct DNA between the duplicated regions. Such a gene disruption can eliminate gene expression if the inserted construct separates the promoter of the gene from the coding region or interrupts the coding sequence such that a non-functional gene product results. A disrupting construct may be simply a selectable marker gene accompanied by 5' and 3' regions homologous to the gene. The selectable marker enables identification of transformants containing the disrupted gene.

The *Bacillus* mutant strains may also be constructed by the process of gene conversion (see, for example, Iglesias and Trautner, Molecular General Genetics 1983, 189, 73-76). For example, in the gene conversion method, a nucleotide sequence corresponding to the gene is mutagenized *in vitro* to produce a defective nucleotide sequence, which is then transformed into the parent *Bacillus* strain to produce a defective gene. By homologous recombination, the defective nucleotide sequence replaces the endogenous gene. It may be desirable that the defective nucleotide sequence also comprises a marker for selection of transformants containing the defective gene.

The *Bacillus* mutant strains may also be constructed by established anti-sense techniques using a nucleotide sequence complementary to the nucleotide sequence of the gene (Parish and Stoker, FEMS Microbiol. Lett. 1997, 154, 151-157). More specifically, expression of the gene by a *Bacillus* strain may be reduced or inactivated by introducing a nucleotide sequence complementary to the nucleotide sequence of the gene, which may be transcribed in the strain and is capable of hybridizing to the mRNA produced in the strain. Under conditions allowing the complementary anti-sense nucleotide sequence to hybridize to the mRNA, the amount of protein translated is thus reduced or eliminated.

The *Bacillus* mutant strains may be further constructed by random or specific mutagenesis using methods well known in the art, including, but not limited to, chemical mutagenesis (see, for example, Hopwood, The Isolation of Mutants in Methods in Microbiology (J.R. Norris and D.W. Ribbons, eds.) pp. 363-433, Academic Press, New York, 1970). Modification of the gene may be performed by subjecting the parent strain to mutagenesis and screening for mutant strains in which expression of the gene has been reduced or inactivated. The mutagenesis, which may be specific or random, may be performed, for example, by use of a suitable physical or chemical mutagenizing agent, use of a suitable oligonucleotide, or subjecting the DNA sequence to PCR generated mutagenesis. Furthermore, the mutagenesis may be performed by use of any combination of these mutagenizing methods.

Examples of a physical or chemical mutagenizing agent suitable for the present purpose include ultraviolet (UV) irradiation, hydroxylamine, N-methyl-N'-nitro-N-nitrosoguanidine (MNNG), N-methyl-N'-nitrosogaunidine (NTG) O-methyl hydroxylamine, nitrous acid, ethyl methane sulphonate (EMS), sodium bisulphite, formic acid, and nucleotide analogues. When such agents are used, the mutagenesis is typically performed by incubating the parent strain to be mutagenized in the presence of the mutagenizing agent of choice under suitable conditions, and selecting for mutants exhibiting reduced or no expression of the gene.

A nucleotide sequence homologous or complementary to a gene described herein may be used from other microbial sources to disrupt the corresponding gene in a *Bacillus* strain of choice.

In one aspect, the modification of a gene in the *Bacillus* mutant is unmarked with a selectable marker. Removal of the selectable marker gene may be accomplished by culturing the mutants on a counter-selection medium. Where the selectable marker gene contains repeats flanking its 5' and 3' ends, the repeats will facilitate the looping out of the selectable marker gene by homologous recombination when the mutant strain is submitted to counter-selection. The selectable marker gene may also be removed by homologous recombination by introducing into the mutant strain a nucleic acid fragment comprising 5' and 3' regions of the defective gene, but lacking the selectable marker gene, followed by selecting on the counter-selection medium. By homologous recombination, the defective gene containing the selectable marker gene is replaced with the nucleic acid fragment lacking the selectable marker gene. Other methods known in the art may also be used.

**Transformed DNA and Related Methods**

The *Bacillus* mutants described herein are useful for producing *Bacillus* transformants. In one aspect is a *Bacillus* mutant comprising a heterologous polynucleotide.

The transformed DNA described herein can be any DNA of interest. The DNA may be of genomic, cDNA, semisynthetic, synthetic origin, or any combinations thereof. The DNA may be a heterologous polynucleotide that encodes any polypeptide having biological activity of interest or may be a DNA involved in the expression of the polypeptide having biological activity, e.g., a promoter.

The polypeptide having a biological activity may be any polypeptide of interest. The polypeptide may be native or foreign to the *Bacillus* host cell of interest. The polypeptide may be naturally occurring allelic and engineered variations of the below-mentioned polypeptides and hybrid polypeptides.

The term "polypeptide" is not meant herein to refer to a specific length of the encoded product and, therefore, encompasses peptides, oligopeptides, and proteins. The term "polypeptide" also encompasses hybrid polypeptides, which comprise a combination of partial or complete polypeptide sequences obtained from at least two different polypeptides wherein one or more may be foreign to the *Bacillus* cell. Polypeptides further include naturally occurring allelic and engineered variations of a polypeptide.

In one aspect, the polypeptide is an antibody, antigen, antimicrobial peptide, enzyme, growth factor, hormone, immunodilator, neurotransmitter, receptor, reporter protein, structural protein, and transcription factor.

In another aspect, the polypeptide is an oxidoreductase, transferase, hydrolase, lyase, isomerase, or ligase. In a most preferred aspect, the polypeptide is an alpha-glucosidase, aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, glucocerebrosidase, alpha-glucosidase, beta-glucosidase, invertase, laccase, lipase, mannosidase, mutanase, oxidase, pectinolytic enzyme, peroxidase, phospholipase, phytase, polyphenoloxidase, proteolytic enzyme, ribonuclease, transglutaminase, urokinase, or xylanase.

In another aspect, the polypeptide is an albumin, collagen, tropoelastin, elastin, or gelatin.

In another aspect, the polypeptide is a hybrid polypeptide, which comprises a combination of partial or complete polypeptide sequences obtained from at least two different polypeptides wherein one or more may be foreign to the *Bacillus* host cell.

In another aspect, the polypeptide is a fused polypeptide in which another polypeptide is fused at the N-terminus or the C-terminus of the polypeptide or fragment thereof. A fused polypeptide is produced by fusing a nucleotide sequence (or a portion thereof) encoding one polypeptide to a nucleotide sequence (or a portion thereof) encoding another polypeptide. Techniques for producing fusion polypeptides are known in the art, and include, ligating the coding sequences encoding the polypeptides so that they are in frame and expression of the fused polypeptide is under control of the same promoter(s) and terminator.

The heterologous polynucleotide encoding a polypeptide of interest may be obtained from any prokaryotic, eukaryotic, or other source. For purposes of the present invention, the term "obtained from" as used herein in connection with a given source shall mean that the polypeptide is produced by the source or by a cell in which a gene from the source has been inserted.

Techniques used to isolate or clone a heterologous polynucleotide encoding a polypeptide of interest are known in the art and include isolation from genomic DNA, preparation from cDNA, or a combination thereof. The cloning of the DNA of interest from such genomic DNA can be effected, e.g., by using the well known polymerase chain reaction (PCR). See, for example, Innis et al., PCR Protocols: A Guide to Methods and Application, Academic Press, New York, 1990. The cloning procedures may involve excision and isolation of a desired nucleic acid fragment comprising the nucleic acid sequence encoding the polypeptide, insertion of the fragment into a vector molecule, and incorporation of the recombinant vector into the *Bacillus* mutant where multiple copies or clones of the nucleic acid sequence will be replicated. The DNA may be of genomic, cDNA, RNA, semisynthetic, synthetic origin, or any combinations thereof.

A heterologous polynucleotide encoding a polypeptide of interest may be manipulated in a variety of ways to provide for expression of the polypeptide in a mutant *Bacillus* strain. Manipulation of the polynucleotide's sequence prior to its insertion into a vector may be desirable or necessary depending on the expression vector. The techniques for modifying polynucleotide sequences utilizing recombinant DNA methods are well known in the art.

A nucleic acid construct comprising a polynucleotide encoding a polypeptide may be operably linked to one or more (e.g., two, several) control sequences capable of directing expression of the coding sequence in a mutant *Bacillus* strain of the present invention under conditions compatible with the control sequences.

The control sequence may be an appropriate promoter sequence, a nucleotide sequence that is recognized by a mutant *Bacillus* strain of the present invention for expression of the polynucleotide encoding the polypeptide. The promoter sequence contains transcriptional control sequences that mediate expression of the polypeptide. The promoter may be any nucleotide sequence that shows transcriptional activity in the mutant *Bacillus* strain, including mutant, truncated, and hybrid promoters, and may be obtained from genes encoding extracellular or intracellular polypeptides either native or foreign to the mutant *Bacillus* strain.

Examples of suitable promoters for directing the transcription of the nucleic acid constructs in a mutant *Bacillus* strain are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *E. coli lac* operon, *E. coli trc* promoter (Egon et al., Gene 1988, 69, 301-315), *Streptomyces coelicolor* agarase gene (*dagA*), and prokaryotic beta-lactamase gene (Villa-Kamaroff et al., Proc. Natl. Acad. Sci. U.S.A. 1978, 75, 3727-3731), as well as the *tac* promoter (DeBoer et al., Proc. Natl. Acad. Sci. U.S.A. 1983, 80, 21-25). Further promoters are described in "Useful proteins from recombinant bacteria" in Gilbert et al., Scientific American 1980, 242, 74-94; and in Sambrook et al., **1989**, *supra.*

The control sequence may also be a suitable transcription terminator sequence, a sequence recognized by a mutant *Bacillus* strain to terminate transcription. The terminator sequence is operably linked to the 3' terminus of the nucleotide sequence encoding the heterologous polypeptide. Any terminator that is functional in a *Bacillus* strain may be used.

The control sequence may also be a suitable leader sequence, a nontranslated region of mRNA that is important for translation by a mutant *Bacillus* strain. The leader sequence is operably linked to the 5' terminus of the nucleotide sequence encoding the heterologous polypeptide. Any leader sequence that is functional in the mutant *Bacillus* strain may be used.

The control sequence may also be a signal peptide coding sequence that codes for an amino acid sequence linked to the amino terminus of a polypeptide and directs the encoded polypeptide into the cell's secretory pathway. The 5' end of the coding sequence of the nucleotide sequence may inherently contain a signal peptide coding sequence naturally linked in translation reading frame with the segment of the coding sequence that encodes the secreted polypeptide. Alternatively, the 5' end of the coding sequence may contain a signal peptide coding sequence that is foreign to the coding sequence. The foreign signal peptide coding sequence may be required where the coding sequence does not naturally contain a signal peptide coding sequence. Alternatively, the foreign signal peptide coding sequence may simply replace the natural signal peptide coding sequence in order to enhance secretion of the polypeptide. However, any signal peptide coding sequence that directs the expressed polypeptide into the secretory pathway of the mutant *Bacillus* strain, i.e., secreted into a culture medium, may be used in the present invention.

A recombinant expression vector comprising a nucleotide sequence, a promoter, and transcriptional and translational stop signals may be used for the recombinant production of a polypeptide of interest. The various nucleic acids and control sequences described herein may be joined together to produce a recombinant expression vector that may include one or more (e.g., two, several) convenient restriction sites to allow for insertion or substitution of the nucleotide sequence encoding the polypeptide at such sites. Alternatively, the nucleotide sequence may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the nucleotide sequence. The choice of the vector will typically depend on its compatibility with the mutant *Bacillus* strain into which the vector is to be introduced. The vector may be a linear or closed circular plasmid.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the mutant *Bacillus* strain, is integrated into the genome and replicated together with the chromosome(s) into which it has been integrated. Furthermore, a single vector or plasmid or two or more vectors or plasmids that together contain the total DNA to be introduced into the genome of the mutant *Bacillus* strain, or a transposon, may be used.

The vector may contain one or more (e.g., two, several) selectable markers that permit easy selection of transformed mutant *Bacillus* strains. A selectable marker is a gene the product of which provides for biocide or viral resistance, resistance to heavy metals, prototrophy to auxotrophs, and the like.

Examples of selectable markers for use in the mutant *Bacillus* strain include, but are not limited to, the *dal* genes from *Bacillus subtilis* or *Bacillus licheniformis*, or markers that confer antibiotic resistance such as ampicillin, chloramphenicol, kanamycin, or tetracycline resistance. Suitable markers for yeast host cells are ADE2, HIS3, LEU2, LYS2, MET3, TRP1, and URA3.

The vectors may contain one or more (e.g., two, several) elements that permit integration of the vector into the *Bacillus* genome or autonomous replication of the vector in the cell independent of the genome.

For integration into the genome of the mutant *Bacillus* strain, the vector may rely on the polynucleotide's sequence encoding the polypeptide of interest or any other element of the vector for integration into the genome by homologous or nonhomologous recombination. Alternatively, the vector may contain additional nucleotide sequences for directing integration by homologous recombination into the genome of the mutant *Bacillus* strain at a precise location(s) in the chromosome. To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which have a high degree of identity to the corresponding target sequence to enhance the probability of homologous recombination. The integrational elements may be any sequence that is homologous with the target sequence in the genome of the mutant *Bacillus* strain. Furthermore, the integrational elements may be non-encoding or encoding nucleotide sequences. On the other hand, the vector may be integrated into the genome of the mutant *Bacillus* strain by non-homologous recombination.

For autonomous replication, the vector may further comprise an origin of replication enabling the vector to replicate autonomously in the mutant *Bacillus* strain. The origin of replication may be any plasmid replicator mediating autonomous replication that functions in a cell. The term "origin of replication" or "plasmid replicator" is defined herein as a nucleotide sequence that enables a plasmid or vector to replicate *in vivo.* Examples of bacterial origins of replication useful in the mutant *Bacillus* strain are the origins of replication of plasmids pBR322, pUC19, pACYC177, and pACYC184 permitting replication in *E. coli,* and pUB110, pE194, pTA1060, and pAMß1 permitting replication in *Bacillus.*

The procedures used to ligate the elements described herein to construct the recombinant expression vectors are well known to one skilled in the art (see, *e.g.*, J. Sambrook, E.F. Fritsch, and T. Maniatus, 1989, Molecular Cloning, A Laboratory Manual, 2d edition, Cold Spring Harbor, New York).

The DNA can also be a control sequence, e.g., promoter, for manipulating the expression of a gene of interest. Non-limiting examples of control sequences are described above.

The DNA can further be a nucleic acid construct for inactivating a gene of interest in a *Bacillus* cell.

The DNA is not to be limited in scope by the specific examples disclosed above, since these examples are intended as illustrations of several aspects of the invention.

Transformation of the DNA into the mutant *Bacillus* strains can be conducted using techniques known in the art, such as electroporation as described in the Examples section below.

The transformants described herein can be isolated using standard techniques well-known in the art, including, but not limited to, streak plate isolation, growth in enrichment or selective media, temperature growth selection, filtration, or single cell isolation techniques, such as flow cytometry and microfluidics.

## Methods of Producing Polypeptides and Fermentation Products

### Polypeptides

As mentioned *supra*, the *Bacillus* mutants described herein can increase the efficiency in producing *Bacillus* transformants which are useful, e.g., in producing a polypeptide having biological activity. Accordingly, in one aspect is a method of producing a polypeptide of interest, comprising: (a) cultivating a *Bacillus* host cell comprising a heterologous polynucleotide encoding the polypeptide of interest under conditions conducive for production of the polypeptide, wherein the *Bacillus* host cell is a *Bacillus* mutant described herein (e.g., a *Bacillus* mutant comprising a disruption to an endogenous *mecA* gene native to the parent *Bacillus* strain and an endogenous *sinI* gene native to the parent *Bacillus* strain); and (b) recovering the polypeptide.

The competent *Bacillus* host cells are cultivated in a nutrient medium suitable for production of a polypeptide of interest using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide of interest to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). The secreted substance of interest, e.g., polypeptide or fermentation product, can be recovered directly from the medium.

The polypeptide having biological activity may be detected using methods known in the art that are specific for the substance. These detection methods may include use of specific antibodies, high performance liquid chromatography, capillary chromatography, formation of an enzyme product, disappearance of an enzyme substrate, or SDS-PAGE. For example, an enzyme assay may be used to determine the activity of a polypeptide having enzyme activity. Procedures for determining enzyme activity are known in the art for many enzymes (see, for example, D. Schomburg and M. Salzmann (eds.), Enzyme Handbook, Springer-Verlag, New York, 1990).

The resulting polypeptide having biological activity may be isolated by methods known in the art. For example, a polypeptide of interest may be isolated from the cultivation medium by conventional procedures including, but not limited to, centrifugation, filtration, extraction, spray-drying, evaporation, or precipitation. The isolated polypeptide may then be further purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF), differential solubility (e.g., ammonium sulfate precipitation), or extraction (see, e.g., Protein Purification, J.-C. Janson and Lars Ryden, editors, VCH Publishers, New York, 1989).

## Examples

Chemicals used as buffers and substrates were commercial products of at least reagent grade.

### Strains

*Escherichia coli*

One Shot™ TOP10 chemically competent *E. coli* cells (Invitrogen, Carlsbad, CA) and Sure™ Competent cells (Stratagene, La Jolla, CA) were used for routine plasmid constructions and propagation.

*B. licheniformis*

*B. licheniformis* SJ1904 (U.S. Patent No. 5,733,753) was used as a host for *mecA* and *sinI* disruption.

### Media

*Bacillus* strains were grown on TBAB (Tryptose Blood Agar Base, Difco Laboratories, Sparks, MD, USA) or LB agar (10 g/l Tryptone, 5 g/l yeast extract, 5 g/l NaCl, 15 g/l agar) plates or in LB liquid medium (10 g/l Tryptone, 5 g/l yeast extract, 5 g/l NaCl).

To select for erythromycin resistance, agar media were supplemented with 1 $\mu$g/ml erythromycin + 25 $\mu$g/ml lincomycin and liquid media were supplemented with 5 $\mu$g/ml erythromycin. To select for spectinomycin resistance, agar media were supplemented with 120 $\mu$g/ml spectinomycin.

Spizizen I medium consists of 1 x Spizizen salts (6 g/l $KH_2PO_4$, 14 g/l $K_2HPO_4$, 2 g/l $(NH_4)_2SO_4$, 1 g/l sodium citrate, 0.2 g/l $MgSO_4$, pH 7.0), 0.5% glucose, 0.1% yeast extract, and 0.02% casein hydrolysate.

Spizizen II medium consists of Spizizen I medium supplemented with 0.5 mM $CaCl_2$, and 2.5 mM $MgCl_2$.

### Example 1: Construction of a *B. licheniformis mecA*-disrupted strain (TaHy9).

Plasmid pBM294 was designed to delete 500 bp within the *B. licheniformis mecA* gene. Genomic DNA was isolated from *B. licheniformis* SJ1904 according the method previously described (Pitcher et. al, Lett. Appl. Microbiol., 1989, 8, 151-156). A 323 bp fragment of the *B. licheniformis* SJ1904 chromosome, including the first 67 bp of the *mecA* coding sequence, was amplified by PCR using primers 0612056 and 0612057 shown below.

Primer 0612056 (SEQ ID NO: 5):

5'-**GAATTC**CATTAATAGCTGCTG-3'

Primer 0612057 (SEQ ID NO: 6):

5'-TCCATACTCTTTCAGCATGGTCTTCGATATCACCGT-3'

A cleavage site for restriction enzyme *EcoR*I (bold) was incorporated into primer 0612056. Primer 0612057 incorporates 18 bp (underlined) corresponding to bp 568 to 588 of the *mecA* coding sequence.

A second 288 bp fragment of the *B. licheniformis* SJ1904 chromosome, including the segment from nucleotides 568 to 639 of the *mecA* coding sequence, was amplified by PCR using primers 0612058 and 0612060 shown below.

Primer 0612058 (SEQ ID NO: 7):

5'-ACGGTGATATCGAAGACCATGCTGAAAGAGTATGGA-3'

Primer 0612060 (SEQ ID NO: 8):

5'-**CTCGAG**CGCATCCTCCCAAAATC-3'

A cleavage site for the *Xho*I restriction enzyme (bold) was incorporated into primer 0612060. Primer 0612058 incorporates 18 bp (underlined) corresponding to bp 47 to 67 of the *mecA* coding sequence. Primers 0612057 and 0612058 are complementary.

The respective DNA fragments were amplified by PCR using the Expand High Fidelity$^{plus}$ PCR system (Roche Diagnostics, Mannheim, Germany). The PCR amplification reaction mixture contained 4 μl (~1 μg) of *B. licheniformis* SJ1904 genomic DNA, 1 μl of sense primer (50 pmol/μl), 1 μl of anti-sense primer (50 pmol/μl), 10 μl of 5X PCR buffer with 15 mM $MgCl_2$, 1 μl of dNTP mix (10 mM each), 32.25 μl water, and 0.75 μl (3.5 U/μl) DNA polymerase mix. An Eppendorf Mastercycler thermocycler was used to amplify the fragment with the following settings: One cycle at 94°C for 2 minutes; 10 cycles each at 94°C for 15 seconds, 58°C for 30 seconds, 72°C for 20 seconds; 15 cycles each at 94°C for 15 seconds, 58°C for 30 seconds, 72°C for 20 seconds plus 5 second elongation at each successive cycle, one cycle at 72°C for 7 minutes; and 4°C hold. The PCR products were purified from a 1.2% agarose (Amresco, Solon, OH) gel with 1x TBE buffer using the Qiagen QIAquick Gel Extraction Kit (Qiagen, Inc., Valencia, CA) according to the manufacturer's instructions.

The purified PCR products were used in a subsequent PCR reaction to create a single fragment using splice overlapping PCR (SOE) using the Expand High Fidelity$^{plus}$ PCR system (Roche Diagnostics) as follows. The PCR amplification reaction mixture contained 2 μl (~50 ng) of gel purified PCR product from primer combination 0612056/0612057, 2 μl (~50 ng) of gel purified PCR product from primer combination 0612058/0612060, 1 μl of primer 0612056 (50 pmol/μl), 1 μl of primer 0612060 (50 pmol/μl), 10 μl of 5X PCR buffer with 15 mM $MgCl_2$, 1 μl of dNTP mix (10 mM each), 32.25 μl water, and 0.75 μl (3.5 U/μl) DNA polymerase mix. An Eppendorf Mastercycler thermocycler was used to amplify the fragment with the following settings: One cycle at 94°C for 2 minutes; 10 cycles each at 94°C for 15 seconds, 58°C for 30 seconds, 72°C for 40 seconds; 15 cycles each at 94°C for 15 seconds, 58°C for 30 seconds, 72°C for 40 seconds plus 5 second elongation at each successive cycle, one cycle at 72°C for 7 minutes; and 4°C hold. The resulting 611 bp PCR product was purified from a 1.2% agarose (Amresco) gel with 1x TBE buffer using the Qiagen QIAquick Gel Extraction Kit (Qiagen, Inc.) according to manufacturer's instructions.

The purified PCR product was cloned into plasmid pCR2.1-TOPO (Invitrogen) according to manufacturer's instructions, resulting is a plasmid designated pBM293. Plasmid pBM293 and plasmid pNNB194 (U.S. Patent No. 5,958,728) were digested with restriction enzymes *Xho*I and *Eco*RI to isolate the 606 bp insert fragment and vector fragment, respectively. These fragments were isolated by 1% agarose gel electrophoresis using TBE buffer followed by purification using the Qiagen QIAquick Gel Extraction Kit (Qiagen, Inc.) according to manufacturer's instructions. The fragments were ligated using a Rapid DNA Ligation Kit following the manufacturer's instructions. A 2 μl aliquot of the ligation was used to transform *E. coli* Sure™ cells according to the manufacturer's instructions. Plasmid DNA was prepared from *E. coli* transformants and digested using restriction enzymes *Eco*RI and *Xho*I, followed by 0.7% agarose gel electrophoresis using TBE buffer and the plasmid identified as having the correct restriction pattern was designated pBM294.

The temperature-sensitive plasmid pBM294 was incorporated into the genome of *B. licheniformis* SJ1904 by chromosomal integration and excision according to the method previously described (U.S. Patent No. 5,843,720). *B. licheniformis* SJ1904 transformants containing plasmid pBM294 were grown on TBAB selective medium at 50°C to force integration of the vector. Desired integrants were chosen based on their ability to grow on TBAB erythromycin/lincomycin selective medium at 50°C. Integrants were then grown without selection in LB medium at 37°C to allow excision of the integrated plasmid. Cells were plated on LB plates and screened for erythromycin-sensitivity.

Genomic DNA was prepared from several erythromycin/lincomycin sensitive isolates above accordingly to the method previously described (Pitcher et. al, *supra*). Genomic PCR confirmed disruption of *mecA* and the resulting strain was designated TaHY9.

**Example 2: Transformation efficiency of a *B. licheniformis mecA*-disrupted strain (TaHy9).**

The *B. licheniformis mecA*-disrupted strain TaHy9 from Example 1 was spread onto LB agar plates to obtain confluent growth after incubation at 37°C overnight. After overnight incubation, approximately 2-3 ml of Spizizen I medium was added to each plate. Cells were scraped using sterile spreaders and transferred into 15 ml Falcon 2059 tubes. Approximately 500 μl of this culture was used to inoculate 50 ml Spizizen I medium containing 1% xylose as the sole carbon source. Growth was monitored using a Klett densitometer. At each cell density corresponding to Klett unit 140, 160, 180, and 200, 250 μl of the culture plus 250 μl Spizizen II medium containing 2 mM EGTA was added to a Falcon 2059 tube. One microgram of transforming DNA (*B. licheniformis* MDT232 chromosomal DNA containing a spectinomycin resistance expression cassette integrated at the *glpD* locus; see WO2008/079895) was added to each tube. Two microliters of 50 μg/ml spectinomycin was also included in the transformation mix. Tubes were incubated at 37°C on a rotational shaker set at 250 rpm for 1 hour. Transformation reactions were plated to LB agar plates containing 120 μg/ml of spectinomycin. Colonies were counted the following day to determine transformation efficiency.

A *B. licheniformis* competent state in a *mecA* disrupted strain was reached during the exponential growth phase and declined as cells entered stationary phase (Figure 7). The highest transformation efficiency was obtained at a Klett densitometer reading of 160 and transformation efficiency declined as cells reached stationary phase.

**Example 3: DNA microarray analysis of a *B. licheniformis mecA*-disrupted strain (TaHy9).**

In order to obtain additional understanding of *B. licheniformis* competence development, DNA microarray technology was used to compare global transcription profiles in the *B. licheniformis* strains TaHy9 (*supra*) and MMar2 (*B. licheniformis* SJ1904 *amyL*::*Xylp-comK*, a *B. licheniformis* strain containing a second copy of the *comK* gene under transcriptional control of the xylose inducible promoter; see US2010/0028944). Strains TaHy9 and MMar2 were grown in triplicate shake flask cultures containing Spizizen I + 1% xylose medium to 160 reading on a Klett densitometer as described in Example 2. RNA samples were purified from triplicate shake flask cultures and DNA microarray analysis was conducted using custom Affymetrix microarray chips (Affymetrix Inc., Santa Clara, CA) designed for use with *B. subtilis* strains 168, A164 and *B. licheniformis* SJ1904. RNA was isolated using the FastRNA™ Pro Blue Kit (MP Biomedicals, LLC, Solon, Ohio), according to manufacturer's recommendations for bacterial RNA isolation. Cells were disrupted two times for 40 seconds at maximum speed (speed 6) in the FastPrep instrument. In addition, 45 μg of RNA from each sample was further purified using in an RNeasy™ column (Qiagen, Inc.) according to manufacturer's specifications. The quality, integrity and concentrations of the RNA samples were measured using a Bioanalyzer instrument (Agilent Technologies Inc., Santa Clara, CA). RNA samples and custom Affymetrix microarray chips were submitted to the UCLA Clinical Microarray Core Research Facility (Los Angeles, California, USA) for labeling, hybridization and scanning. Microarray data were analyzed using a custom script as previously described (Gillespie et al., BMC Research Notes 2010, 3(81)). Data were normalized using Robust Multi-array Average (RMA) (Irizarry et al., Biostatistics 2003, 4, 249-264) followed by differential expression analysis using Linear Models for Microarray Data (Limma) software (Smyth. 2005. Limma: Linear models for microarray data. In: Bioinformatics and Computational Biology Solutions using R and Bioconductor, Springer, New York, pp. 397-420).

The microarray data revealed significantly increased transcript levels for *epsH*, *tapA*, *sigW* and *tasA* genes in *Bacillus licheniformis* strain TaHy9 compared to strain MMar2 ($p < 0.05$).

**Example 4: Construction of a *B. licheniformis mecA*-disrupted + *sinI*-disrupted strain (BaC0155).**

In light of the microarray data of Example 3, a *B. licheniformis mecA sinI* double mutant was constructed to test for improved transformation efficiency.

A plasmid designated pBM317, containing an *oriT* DNA sequence, was constructed to serve as an *E. coli*/*Bacillus* shuttle vector when using conjugation to introduce plasmid DNA from a *Bacillus* host strain to a *Bacillus* recipient strain. The *oriT* DNA segment was PCR amplified from plasmid pSHV002 (US 5,891,701) using primers 1200090 and 1200091 below.

Primer 1200090 (SEQ ID NO: 9):

5'-GATAGCTTGGA GTTTCTAGAG CGGCCGCATT ATTAATCTGT TCAGC-3'

Primer 1200091 (SEQ ID NO: 10):

5'-GAGCTCCACC GCGGTGGCGG CCGCTGCCTT TTAGTCCAGC TG-3'

The 608 bp *oriT* DNA fragment was PCR amplified using the Expand High Fidelity$^{plus}$ PCR system (Roche Diagnostics) with a reaction mixture containing 1 μl (0.113 ng/μl) pSHV002 plasmid DNA, 1 μl primer 1200090 (50 pmol/μl), 1 μl primer 1200091 (50 pmol/μl), 10 μl of 5X PCR buffer with 15 mM $MgCl_2$, 1 μl of dNTP mix (10 mM each), 35.25 μl water, and 0.75 μl (3.5 U/μl) DNA polymerase mix. An Eppendorf Mastercycler thermocycler was used to amplify the fragment with the following settings: One cycle at 94°C for 2 minutes; 10 cycles each at 94°C for 15 seconds, 58°C for 30 seconds, 72°C for 30 seconds; 15 cycles each at 94°C for 15 seconds, 58°C for 30 seconds, 72°C for 30 seconds plus 5 second elongation at each successive cycle, one cycle at 72°C for 7 minutes; and 4°C hold. The resulting PCR product was purified using the Qiagen QIAquick PCR Purification Kit (Qiagen, Inc.) according to the manufacturer's instructions.

The 608 bp PCR amplified *oriT* fragment was cloned into plasmid pNNB194 (U.S. Patent No. 5,958,728) which had been previously digested with restriction enzyme *Not*I using Clontech In-Fusion HD Cloning System (Clontech laboratories, Inc., Mountain View, CA) according to the manufacturer's instructions. A 2 μl aliquot of the In-fusion mix was used to transform *E. coli* Stellar™ cells according to the manufacturer's instructions. Plasmid DNA was prepared from *E. coli* transformants and digested using restriction enzyme *Not*I, followed by 1.0% agarose gel electrophoresis using TBE buffer. The plasmid identified as having the correct restriction pattern was designated pBM317.

Plasmid pBM319 was designed to delete the entire 177 bp *sinI* open reading frame. Genomic DNA was isolated from *B. licheniformis* SJ1904 according to the method previously described (Pitcher et. al, *supra).* A 434 bp fragment of the *B. licheniformis* SJ1904 chromosome, flanking the *sinI* coding sequence at the 5' end, was amplified by PCR from *B. licheniformis* SJ1904 genomic DNA using primers 1200749 and 1200750 shown below.

Primer 1200749 (SEQ ID NO: 11):

5'-**GAATTC**GCCAGAAGCGAACCGC-3'

Primer 1200750 (SEQ ID NO: 12):

5'-CCTTCCTTTC GATATTATAG CACATACATT TCCCCCCCAA AATACTTGAT-3'

A cleavage site for restriction enzyme *Eco*RI (bold) was incorporated into primer 1200749.

A second 430 bp fragment of the *B. licheniformis* SJ1904 chromosome, flanking the *sinI* coding sequence at the 3' end was amplified by PCR from *B. licheniformis* SJ1904 genomic DNA using primers 1200751 and 1200752 shown below.

Primer 1200751 (SEQ ID NO: 13):

5'-ATCAAGTATT TTGGGGGGGA AATGTATGTG CTATAATATC GAAAGGAAGG-3'

Primer 1200752 (SEQ ID NO: 14):

5'-**CTCGAG**AAATGCCAAAGGGAG-3'

A cleavage site for restriction enzyme *Xho*I was incorporated into primer 1200752. Primers 1200750 and 1200751 are complementary.

The respective gene fragments were amplified by PCR using the Expand High Fidelity$^{plus}$ PCR system (Roche Diagnostics) with a reaction mixture containing 4 μl (~1 μg) of *B. licheniformis* SJ1904 genomic DNA, 1 μl of sense primer (50 pmol/μl), 1 μl of anti-sense primer (50 pmol/μl), 10 μl of 5X PCR buffer with 15 mM $MgCl_2$, 1 μl of dNTP mix (10 mM each), 32.25 μl water, and 0.75 μl (3.5 U/μl) DNA polymerase mix. An Eppendorf Mastercycler thermocycler was used to amplify the fragment with the following settings: One cycle at 94°C for 2 minutes; 10 cycles each at 94°C for 15 seconds, 58°C for 30 seconds, 72°C for 20 seconds; 15 cycles each at 94°C for 15 seconds, 58°C for 30 seconds, 72°C for 20 seconds plus 5 second elongation at each successive cycle, one cycle at 72°C for 7 minutes; and 4° C hold. The PCR products were purified from a 1.0% agarose (Amresco) gel with 1x TBE buffer using the Qiagen QIAquick Gel Extraction Kit (Qiagen, Inc.) according to the manufacturer's instructions.

The purified PCR products were used in a second PCR reaction to create a single fragment using splice overlapping PCR (SOE) using the Expand High Fidelity$^{plus}$ PCR system (Roche Diagnostics) as follows. The PCR amplification reaction mixture contained 2 μl of gel purified PCR product from primer combination 1200749/1200750, 2 μl of gel purified PCR product from primer combination 1200751/1200752, 1 μl of primer 0612056 (50 pmol/μl), 1 μl of primer 0612060 (50 pmol/μl), 10 μl of 5X PCR buffer with 15 mM $MgCl_2$, 1 μl of dNTP mix (10 mM each), 32.25 μl water, and 0.75 μl (3.5 U/μl) DNA polymerase mix. An Eppendorf Mastercycler thermocycler was used to amplify the fragment with the following settings: One cycle at 94°C for 2 minutes; 10 cycles each at 94°C for 15 seconds, 58°C for 30 seconds, 72°C for 40 seconds; 15 cycles each at 94°C for 15 seconds, 58°C for 30 seconds, 72°C for 40 seconds plus 5 second elongation at each successive cycle, one cycle at 72°C for 7 minutes; and 4°C hold. The resulting 814 bp PCR product was purified from a 1.0% agarose (Amresco) gel with 1x TBE buffer using the Qiagen QIAquick Gel Extraction Kit (Qiagen, Inc.) according to the manufacturer's instructions.

The purified PCR product was cloned into plasmid pCR2.1-TOPO (Invitrogen) according to the manufacturer's instructions. The resulting plasmid and plasmid pBM317 were digested with restriction enzymes *Xho*I and *Eco*RI to isolate the 808 bp insert fragment and vector fragment, respectively. These fragments were isolated by 1% agarose gel electrophoresis using TBE buffer followed by purification using the Qiagen QIAquick Gel Extraction Kit (Qiagen, Inc.) according to manufacturer's instructions. The fragments were ligated using a Rapid DNA Ligation Kit following the manufacturer's instructions. A 2 μl aliquot of the ligation was used to transform *E. coli* Stellar™ cells according to the manufacturer's instructions. Plasmid DNA was prepared from *E. coli* transformants and digested using restriction enzymes *Eco*RI and *Xho*I, followed by 0.7% agarose gel electrophoresis using TBE buffer. The plasmid identified as having the correct restriction pattern was designated pBM319.

Plasmid pBM319 was incorporated into the genome of *B. licheniformis* SJ1904 by chromosomal integration and excision of the temperature-sensitive plasmid pBM319. *B. licheniformis* SJ1904 transformants containing plasmid pBM294 were grown on TBAB selective medium at 50°C to force integration of the vector. Desired integrants were chosen based on their ability to grow on TBAB erythromycin/lincomycin selective medium at 50°C. Integrants were then grown without selection in LB medium at 37°C to allow excision of the integrated plasmid. Cells were plated on LB plates and screened for erythromycin-sensitivity. Genomic DNA was prepared from several erythromycin/lincomycin sensitive isolates according to the method previously described (Pitcher et. al, *supra*). Genomic PCR confirmed disruption of the *mecA* gene. The resulting strain was designated BaC0155.

### Example 5: Transformation efficiency of a *B. licheniformis mecA*-disrupted + *sinI*-disrupted strain (BaC0155).

*B. licheniformis* strains TaHy9 and BaC0155 were grown in Spizizen I medium and transformed with genomic DNA (*B. licheniformis* MDT232 chromosomal DNA containing a spectinomycin resistance expression cassette integrated at the *glpD* locus; see WO2008/079895), as described *supra.* The results of two independent experiments are shown in Figure 8. Based on these experiments, disruption of both the *sinI* gene and the *mecA* gene (strain BaC0155) roughly doubles the transformation efficiency when compared to disruption of the *mecA* gene alone (strain TaHy9).

Although the foregoing has been described in some detail by way of illustration and example for the purposes of clarity of understanding, it is apparent to those skilled in the art that any equivalent aspect or modification may be practiced. Therefore, the description and examples should not be construed as limiting the scope of the invention.

**Claims**

1. An isolated mutant *Bacillus* strain, comprising disruption to an endogenous *mecA* gene native to the parent *Bacillus* strain and disruption to an endogenous *sinl* gene native to the parent *Bacillus* strain, wherein the endogenous *mecA* gene native to the parent *Bacillus* strain encodes for a polypeptide having at least 80% sequence identity to SEQ ID NO: 2 or 16, wherein the endogenous *sinl* gene native to the parent *Bacillus* strain encodes for a polypeptide having at least 80% sequence identity to SEQ ID NO: 4 or 18, and wherein the mutant is capable of producing at least 10-fold more transformants compared to the parent *Bacillus* strain that lacks disruption to the endogenous *mecA* gene and lacks disruption to the endogenous *sinl* gene, when cultivated under identical conditions.

2. The isolated mutant of claim 1, wherein the *Bacillus* strain is selected from *Bacillus alkalophilus*, *Bacillus amyloliq-*

*uefaciens*, *Bacillus brevis, Bacillus circulans*, *Bacillus clausii, Bacillus coagulans*, *Bacillus firmus, Bacillus lautus*, *Bacillus lentus*, *Bacillus licheniformis*, *Bacillus megaterium*, *Bacillus pumilus*, *Bacillus stearothermophilus*, *Bacillus subtilis*, or *Bacillus thuringiensis.*

**3.** The isolated mutant of any of claims 1-2, wherein the *Bacillus* strain is a *Bacillus licheniformis* strain.

**4.** The isolated mutant of any of claims 1-3, wherein the *Bacillus* strain is a *Bacillus subtilis* strain.

**5.** The mutant of any one of claims 1-4, wherein the endogenous *mecA* gene encodes a polypeptide comprising or consisting of SEQ ID NO: 2 or 16.

**6.** The mutant of any one of claims 1-5, wherein the coding sequence of the endogenous *mecA* gene comprises or consists of SEQ ID NO: 1 or 15.

**7.** The mutant of any one of claims 1-6, wherein disruption to the endogenous *mecA* gene occurs in the coding sequence and/or promoter sequence.

**8.** The mutant of any one of claims 1-7, wherein the endogenous *sinl* gene encodes a polypeptide comprising or consisting of SEQ ID NO: 4 or 18.

**9.** The mutant of any one of claims 1-8, wherein the coding sequence of the endogenous *sinl* gene comprises or consists of SEQ ID NO: 3 or 17.

**10.** The mutant of any one of claims 1-9, wherein disruption to the endogenous *sinl* gene occurs in the coding sequence and/or promoter sequence.

**11.** The mutant of any one of claims 1-10, further comprising a heterologous polynucleotide encoding a polypeptide of interest.

**12.** The mutant of claim 11, wherein the polypeptide of interest is an oxidoreductase, transferase, hydrolase, lyase, isomerase, or ligase.

**13.** The mutant of claim 11, wherein the polypeptide of interest is an alpha-glucosidase, aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, glucocerebrosidase, alpha-glucosidase, beta-glucosidase, invertase, laccase, lipase, mannosidase, mutanase, oxidase, pectinolytic enzyme, peroxidase, phospholipase, phytase, polyphenoloxidase, proteolytic enzyme, ribonuclease, transglutaminase, urokinase, or xylanase.

**14.** A method of producing a polypeptide of interest, comprising:

(a) cultivating the isolated *Bacillus* mutant of any of claims 11-13; and
(b) recovering the polypeptide of interest.

**Patentansprüche**

**1.** Isolierter mutierter *Bacillus*-Stamm, umfassend eine Unterbrechung eines endogenen *mecA*-Gens, das bezüglich des parentalen *Bacillus*-Stamms nativ ist, und eine Unterbrechung eines endogenen *sinl*-Gens, das bezüglich des parentalen *Bacillus*-Stamms nativ ist, wobei das endogene *mecA*-Gen, das bezüglich des parentalen *Bacillus*-Stamms nativ ist, ein Polypeptid mit mindestens 80% Sequenzidentität zu SEQ ID NO: 2 oder 16 kodiert, wobei das endogene *sinl*-Gen, das bezüglich des parentalen *Bacillus*-Stamms nativ ist, ein Polypeptid mit mindestens 80% Sequenzidentität zu SEQ ID NO: 4 oder 18 kodiert, und wobei die Mutante zum Herstellen von mindestens 10-fach mehr Transformanten fähig ist verglichen zum parentalen *Bacillus*-Stamm, dem eine Unterbrechung des endogenen *mecA*-Gens fehlt und dem eine Unterbrechung des endogenen *sinl*-Gens fehlt, wenn sie unter identischen Bedingungen kultiviert werden.

**2.** Isolierte Mutante nach Anspruch 1, wobei der *Bacillus*-Stamm ausgewählt ist aus *Bacillus alkalophilus*, *Bacillus*

*amyloliquefaciens*, *Bacillus brevis*, *Bacillus circulans*, *Bacillus clausii*, *Bacillus coagulans*, *Bacillus firmus, Bacillus lautus*, *Bacillus lentus*, *Bacillus licheniformis, Bacillus megaterium*, *Bacillus pumilus*, *Bacillus stearothermophilus*, *Bacillus subtilis* oder *Bacillus thuringiensis.*

**3.** Isolierte Mutante nach einem beliebigen der Ansprüche 1-2, wobei der *Bacillus-Stamm* ein *Bacillus licheniformis*-Stamm ist.

**4.** Isolierte Mutante nach einem beliebigen der Ansprüche 1-3, wobei der *Bacillus*-Stamm ein *Bacillus subtilis*-Stamm ist.

**5.** Mutante nach einem beliebigen der Ansprüche 1-4, wobei das endogene *mecA*-Gen ein Polypeptid kodiert, das SEQ ID NO: 2 oder 16 umfasst oder daraus besteht.

**6.** Mutante nach einem beliebigen der Ansprüche 1-5, wobei die kodierende Sequenz des endogenen *mecA*-Gens SEQ ID NO: 1 oder 15 umfasst oder daraus besteht.

**7.** Mutante nach einem beliebigen der Ansprüche 1-6, wobei eine Unterbrechung des endogenen *mecA*-Gens in der kodierenden Sequenz und/oder Promotorsequenz auftritt.

**8.** Mutante nach einem beliebigen der Ansprüche 1-7, wobei das endogene *sinl*-Gen ein Polypeptid kodiert, das SEQ ID NO: 4 oder 18 umfasst oder daraus besteht.

**9.** Mutante nach einem beliebigen der Ansprüche 1-8, wobei die kodierende Sequenz des endogenen *sinl*-Gens SEQ ID NO: 3 oder 17 umfasst oder daraus besteht.

**10.** Mutante nach einem beliebigen der Ansprüche 1-9 wobei eine Unterbrechung des endogenen *sinl*-Gens in der kodierenden Sequenz und/oder Promotorsequenz auftritt.

**11.** Mutante nach einem beliebigen der Ansprüche 1-10, die weiterhin ein heterologes Polynukleotid umfasst, das ein Polypeptid von Interesse kodiert.

**12.** Mutante nach Anspruch 11, wobei das Polypeptid von Interesse eine Oxidoreduktase, Transferase, Hydrolase, Lyase, Isomerase oder Ligase ist.

**13.** Mutante nach Anspruch 11, wobei das Polypeptid von Interesse eine alpha-Glucosidase, Aminopeptidase, Amylase, Carbohydrase, Carboxypeptidase, Katalase, Cellulase, Chitinase, Cutinase, Cyclodextringlycosyltransferase, Desoxyribonuclease, Esterase, alpha-Galactosidase, beta-Galactosidase, Glucoamylase, Glucocerebrosidase, alpha-Glucosidase, beta-Glucosidase, Invertase, Laccase, Lipase, Mannosidase, Mutanase, Oxidase, pektinolytisches Enzym, Peroxidase, Phospholipase, Phytase, Polyphenoloxidase, proteolytisches Enzym, Ribonuclease, Transglutaminase, Urokinase oder Xylanase ist.

**14.** Verfahren zum Herstellen eines Polypeptids von Interesse, umfassend:

a) Kultivieren der isolierten *Bacillus*-Mutante gemäß einem beliebigen der Ansprüche 11-13; und
b) Gewinnen des Polypeptids von Interesse.

**Revendications**

**1.** Souche mutante isolée de *Bacillus,* comprenant une disruption d'un gène *mecA* endogène natif de la souche de *Bacillus* parente et une disruption d'un gène *sinl* endogène natif de la souche de *Bacillus* parente, dans laquelle le gène *mecA* endogène natif de la souche de *Bacillus* parente code pour un polypeptide présentant une identité de séquence d'au moins 80 % avec SEQ ID No: 2 ou 16, dans laquelle le gène *sinl* endogène natif de la souche de *Bacillus* parente code pour un polypeptide présentant une identité de séquence d'au moins 80 % avec la SEQ ID No: 4 ou 18, et dans laquelle le mutant est capable de produire au moins 10 fois plus de transformants comparativement à la souche de *Bacillus* parente qui ne présente pas de disruption du gène *mecA* endogène et ne présente pas de disruption du gène *sinl* endogène, quand ils sont cultivés dans des conditions identiques.

**2.** Mutant isolé selon la revendication 1, dans lequel la souche de *Bacillus* est choisie parmi *Bacillus alkalophilus*, *Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* ou *Bacillus thuringiensis.*

**3.** Mutant isolé selon l'une quelconque des revendications 1 à 2, dans lequel la souche de *Bacillus* est une souche de *Bacillus licheniformis.*

**4.** Mutant isolé selon l'une quelconque des revendications 1 à 3, dans lequel la souche de *Bacillus* est une souche de *Bacillus subtilis.*

**5.** Mutant selon l'une quelconque des revendications 1 à 4, dans lequel le gène *mecA* endogène code pour un polypeptide comprenant ou constitué de la SEQ ID No: 2 ou 16.

**6.** Mutant selon l'une quelconque des revendications 1 à 5, dans lequel la séquence codante du gène *mecA* endogène comprend ou est constituée de la SEQ ID No: 1 ou 15.

**7.** Mutant selon l'une quelconque des revendications 1 à 6, dans lequel la disruption du gène *mecA* endogène se produit dans la séquence codante et/ou la séquence du promoteur.

**8.** Mutant selon l'une quelconque des revendications 1 à 7, dans lequel le gène *sinl* endogène code pour un polypeptide comprenant ou constitué de la SEQ ID No: 4 ou 18.

**9.** Mutant selon l'une quelconque des revendications 1 à 8, dans lequel la séquence codante du gène *sinl* endogène comprend ou est constituée de la SEQ ID No: 3 ou 17.

**10.** Mutant selon l'une quelconque des revendications 1 à 9, dans lequel la disruption du gène *sinl* endogène se produit dans la séquence codante et/ou la séquence du promoteur.

**11.** Mutant selon l'une quelconque des revendications 1 à 10, comprenant en outre un polynucléotide hétérologue codant pour un polypeptide d'intérêt.

**12.** Mutant selon la revendication 11, dans lequel le polypeptide d'intérêt est une oxydoréductase, une transférase, une hydrolase, une lyase, une isomérase, ou une ligase.

**13.** Mutant selon la revendication 11, dans lequel le polypeptide d'intérêt est une alpha-glucosidase, une aminopeptidase, une amylase, une carbohydrase, une carboxypeptidase, une catalase, une cellulase, une chitinase, une cutinase, une cyclodextrine glycosyltransférase, une désoxyribonucléase, une estérase, une alpha-galactosidase, une bêta-galactosidase, une glucoamylase, une glucocérébrosidase, une alpha-glucosidase, une bêta-glucosidase, une invertase, une laccase, une lipase, une mannosidase, une mutanase, une oxydase, une enzyme pectinolytique, une peroxydase, une phospholipase, une phytase, une polyphénoloxydase, une enzyme protéolytique, une ribonucléase, une transglutaminase, une urokinase, ou une xylanase.

**14.** Méthode de production d'un polypeptide d'intérêt, comprenant :

(a) la culture du mutant de *Bacillus* isolé selon l'une quelconque des revendications 11 à 13 ; et
(b) la récupération du polypeptide d'intérêt.

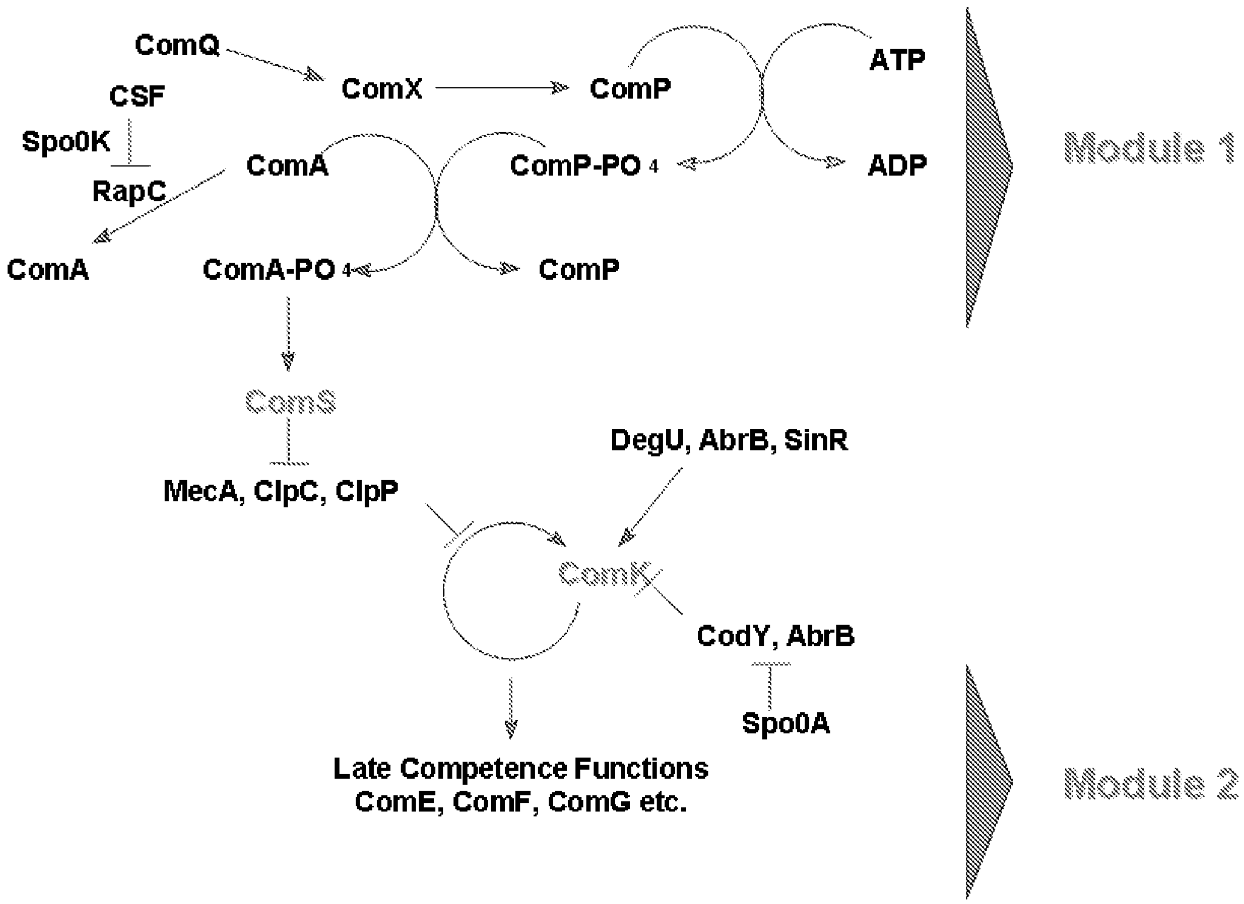
ComQ
CSF
Spo0K
RapC
ComX
ComP
ATP
ComA
ComP-PO 4
ADP
Module 1
ComA
ComA-PO 4
ComP
ComS
MecA, ClpC, ClpP
DegU, AbrB, SinR
ComK
CodY, AbrB
Spo0A
Late Competence Functions
ComE, ComF, ComG etc.
Module 2

Fig. 1

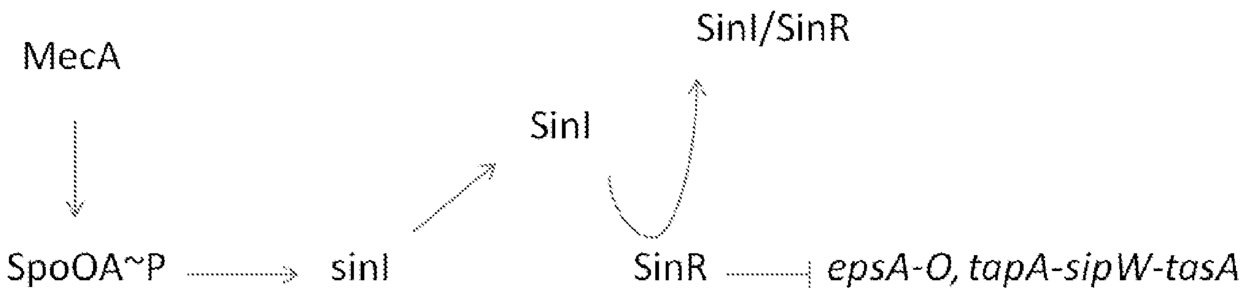


Fig. 2

```
     M  E  I  E  R  I  N  E  H  T  V  K  F  Y  I  S  Y  G  D  I  E
  1 ATGGAAATCGAAAGAATAAACGAACACACGGTTAAGTTTTATATTTCCTACGGTGATATCGAA
     D  R  G  F  D  R  E  E  I  W  Y  N  R  E  R  S  E  E  L  F  W
 64 GACCGCGGGTTTGACAGAGAAGAAATTTGGTACAATCGAGAACGCAGTGAAGAGCTTTTTTGG
     E  M  M  D  E  V  H  E  E  E  E  F  A  V  E  G  P  L  W  I  Q
127 GAAATGATGGACGAAGTGCACGAAGAAGAAGAGTTTGCCGTTGAAGGCCCCCTTTGGATTCAA
     V  Q  A  L  D  K  G  L  E  I  I  V  T  R  A  Q  L  S  K  D  G
190 GTGCAGGCCCTTGACAAGGGATTGGAAATCATTGTGACAAGAGCTCAGTTGTCCAAAGACGGA
     Q  K  L  E  L  P  I  P  E  D  K  K  Q  H  V  A  E  E  S  L  D
253 CAAAAGCTGGAACTGCCGATTCCTGAAGATAAAAAACAGCATGTAGCAGAAGAAAGCCTTGAT
     A  L  L  D  D  F  Q  K  E  E  Q  A  E  E  Q  K  L  Q  F  V  L
316 GCTTTGCTTGACGACTTTCAAAAAGAAGAGCAGGCAGAAGAGCAGAAACTGCAGTTTGTTTTA
     K  F  D  D  F  E  D  L  I  S  L  S  K  M  S  V  S  G  C  Q  T
379 AAATTTGACGATTTTGAGGATTTAATCTCACTGTCAAAAATGTCTGTCAGCGGTTGCCAAACG
     T  L  Y  S  H  E  N  R  Y  Y  L  F  V  D  F  N  E  L  P  D  E
442 ACATTGTACTCTCATGAAAACCGCTATTATTTATTCGTGGATTTCAATGAACTGCCTGATGAA
     E  V  E  N  Q  L  S  I  L  L  E  Y  A  S  E  S  K  M  T  I  H
505 GAGGTGGAAAACCAGCTGAGCATTCTGCTGGAATATGCCTCAGAATCAAAGATGACGATTCAT
     M  L  K  E  Y  G  K  L  I  A  A  D  H  A  L  H  T  I  K  K  H
568 ATGCTGAAAGAGTATGGAAAACTGATTGCAGCGGATCATGCTCTTCATACAATAAAAAAGCAC
     F  A  *
631 TTTGCATAA
```

Fig. 3

```
    M  N  K  D  K  N  E  K  E  E  L  D  E  E  W  T  D  L  I  K  H
  1 ATGAATAAAGATAAAAATGAGAAAGAAGAATTGGATGAGGAGTGGACAGACTTGATTAAACAC
    A  L  E  Q  G  I  S  P  E  E  I  R  I  F  L  N  L  G  K  K  S
 64 GCTCTTGAACAAGGCATTAGTCCAGAGGAAATACGTATTTTTCTCAATTTGGGAAAGAAGTCT
    S  N  P  S  T  S  I  E  R  S  H  S  I  N  P  F  *
127 TCAAATCCTTCCACATCAATTGAAAGAAGTCATTCAATAAATCCTTTCTGA
```

Fig. 4

```
     M  E  I  E  R  I  N  E  H  T  V  K  F  Y  M  S  Y  G  D  I  E
  1 ATGGAAATTGAAAGAATTAACGAGCATACAGTAAAATTTTATATGTCTTACGGAGATATTGAA
     D  R  G  F  D  R  E  E  I  W  Y  N  R  E  R  S  E  E  L  F  W
 64 GATCGCGGTTTTGACAGAGAAGAAATTTGGTATAACCGTGAGCGCAGTGAAGAACTTTTCTGG
     E  V  M  D  E  V  H  E  E  E  E  F  A  V  E  G  P  L  W  I  Q
127 GAAGTCATGGATGAAGTTCATGAAGAAGAGGAATTCGCTGTGGAAGGCCCACTTTGGATTCAA
     V  Q  A  L  D  K  G  L  E  I  I  V  T  K  A  Q  L  S  K  D  G
190 GTTCAGGCACTGGACAAAGGATTAGAAATCATCGTCACAAAAGCCCAGCTTTCCAAAGACGGA
     Q  K  L  E  L  P  I  P  E  D  K  K  Q  E  P  A  S  E  D  L  D
253 CAAAAGCTCGAACTGCCGATTCCTGAGGATAAAAAGCAAGAACCAGCATCTGAGGATCTTGAC
     A  L  L  D  D  F  Q  K  E  E  Q  A  V  N  Q  E  E  K  E  Q  K
316 GCCTTGCTGGATGATTTCCAGAAAGAAGAGCAAGCCGTCAATCAGGAAGAGAAGGAGCAAAAG
     L  Q  F  V  L  R  F  G  D  F  E  D  V  I  S  L  S  K  L  N  V
379 CTTCAATTTGTCTTGCGATTTGGCGATTTTGAGGATGTTATTTCTCTATCTAAATTGAACGTT
     N  G  S  K  T  T  L  Y  S  F  E  N  R  Y  Y  L  Y  V  D  F  C
442 AACGGAAGCAAAACGACTTTATATTCGTTTGAGAACCGATATTATTTATATGTAGATTTTTGC
     N  M  T  D  E  E  V  E  N  Q  L  S  I  L  L  E  Y  A  T  E  S
505 AATATGACTGATGAAGAGGTTGAAAATCAGCTAAGCATCCTGCTGGAGTACGCAACTGAATCC
     S  I  S  I  H  R  L  E  E  Y  G  K  L  I  I  S  E  H  A  L  E
568 TCAATCAGCATACACCGTCTTGAAGAGTACGGCAAGCTGATTATTTCAGAGCATGCTCTAGAA
     T  I  K  K  H  F  A  S  *
631 ACGATAAAAAAACACTTTGCATCATAG
```

Fig. 5

```
    M  K  N  A  K  Q  E  H  F  E  L  D  Q  E  W  V  E  L  M  V  E
  1 ATGAAGAATGCAAAACAAGAGCACTTTGAATTGGATCAAGAATGGGTTGAATTAATGGTGGAA
    A  K  E  A  N  I  S  P  E  E  I  R  K  Y  L  L  L  N  K  K  S
 64 GCCAAAGAGGCAAATATCAGCCCGGAAGAAATACGAAAATATTTACTTTTAAACAAAAAGTCT
    A  H  P  G  P  A  A  R  S  H  T  V  N  P  F  *
127 GCTCATCCTGGTCCGGCAGCCAGAAGTCATACCGTAAATCCTTTCTGA
```

Fig. 6

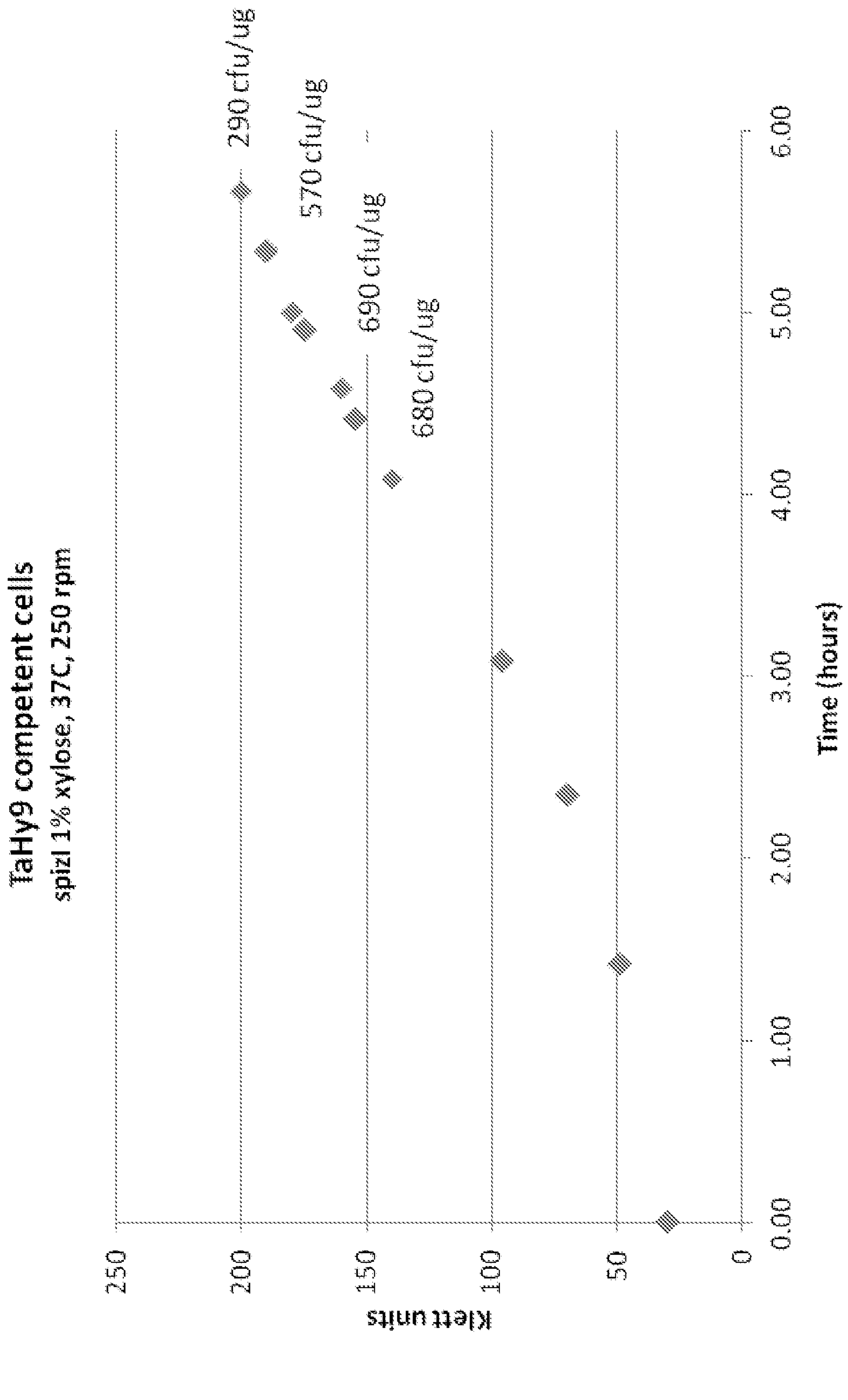
TaHy9 competent cells
spizI 1% xylose, 37C, 250 rpm
Klett units
0
50
100
150
200
250
0.00
1.00
2.00
3.00
4.00
5.00
6.00
Time (hours)
290 cfu/ug
570 cfu/ug
690 cfu/ug
680 cfu/ug

Fig. 7

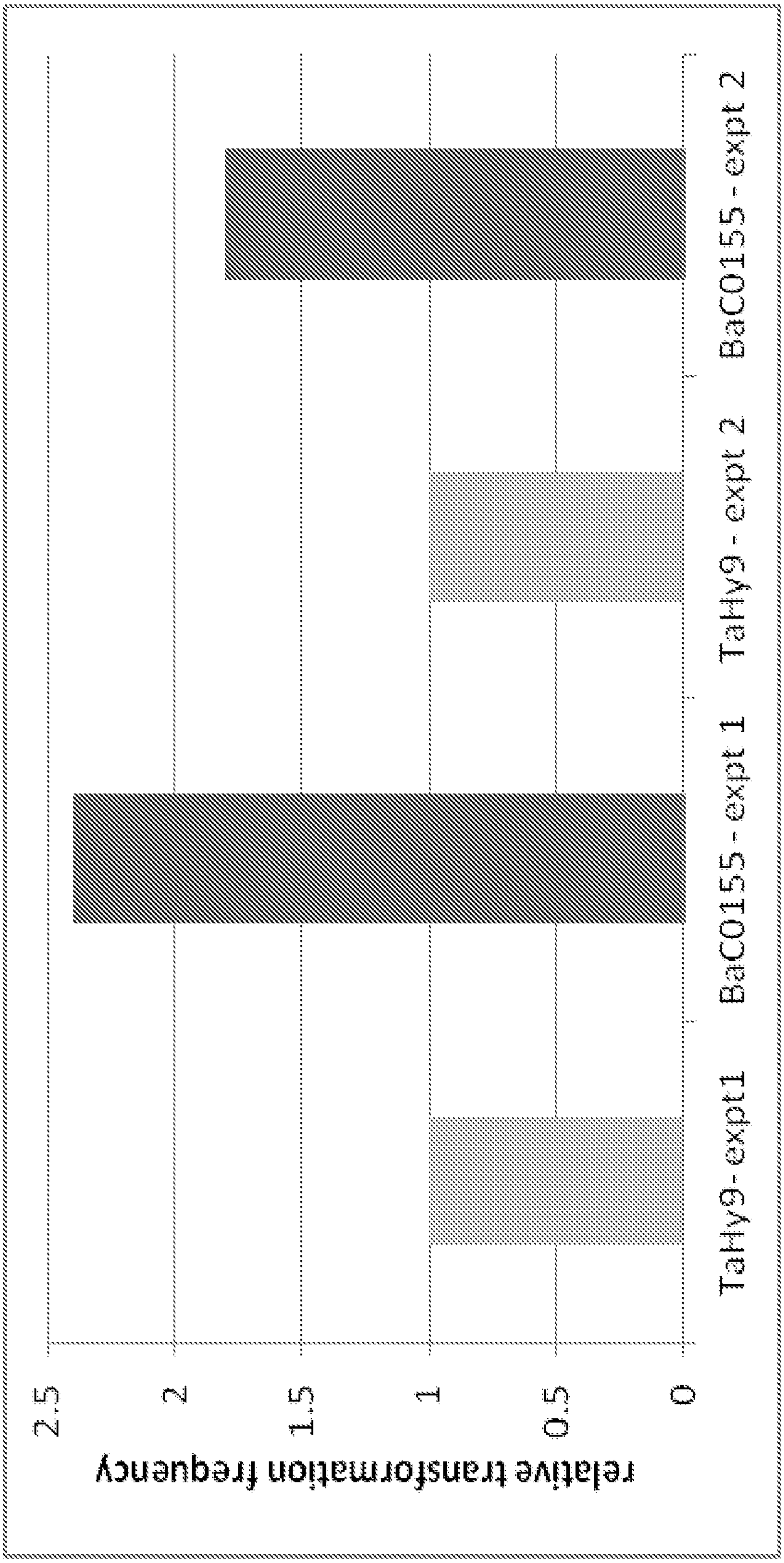
relative transformation frequency
2.5
2
1.5
1
0.5
0
TaHy9 - expt1
BaC0155 - expt 1
TaHy9 - expt 2
BaC0155 - expt 2

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100028944 A
- WO 9517413 A
- WO 9522625 A
- US 5223409 A
- WO 9206204 A
- US 5733753 A
- US 5958728 A
- US 5843720 A
- WO 2008079895 A
- US 5891701 A
- US 61706661 B


### Non-patent literature cited in the description


- **BERKA et al.** *Mol. Microbiol.,* 2002, vol. 43, 1331-1345
- **DUBNAU.** *Annual Rev. Microbiol.,* 1999, vol. 53, 217-244
- **AVERY.** *Trends Microbiol.,* 2005, vol. 13, 459-462
- **BLATTNER et al.** *Science,* 1997, vol. 277, 1453-1474
- **HAYASHI et al.** *Mol. Syst. Biol.,* 2006
- **PERNA et al.** *Nature,* 2001, vol. 409, 529-533
- **WELCH et al.** *Proc. Natl. Acad Sci. USA,* 2002, vol. 99, 17020-17024
- **BRZUSZKIEWICZ et al.** *Proc. Natl. Acad. Sci. USA,* 2006, vol. 103, 12879-12884
- **TURGAY et al.** *EMBO J.,* 1998, vol. 17, 6730-6738
- **HAHN.** *Mol. Microbiol.,* 1995, vol. 18, 755-767
- **PREPIAK.** *Mol. Microbiol.,* 2011, vol. 80, 1014-1030
- Methods in Yeast Genetics. Cold Spring Harbor Press, 1997
- **STAHL ; FERRARI.** *J. Bacteriol.,* 1984, vol. 158, 411-418
- **ANAGNOSTOPOULOS ; SPIZIZEN.** *J. Bacteriol.,* 1961, vol. 81, 741-746
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet,* 2000, vol. 16, 276-277
- **RICE et al.** *EMBOSS: The European Molecular Biology Open Software Suite,* 2000
- **BOLTON ; MCCARTHY.** *Proc. Natl. Acad. Sci. USA,* 1962, vol. 48, 1390
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. U.S.A.,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnol.,* 1999, vol. 17, 893-896
- **BOTSTEIN ; SHORTLE.** *Science,* 1985, vol. 229, 4719
- **LO et al.** *Proc. Natl. Acad. Sci. U.S.A.,* 1985, vol. 81, 2285
- **HIGUCHI et al.** *Nucleic Acids Res,* 1988, vol. 16, 7351
- **SHIMADA.** *Meth. Mol. Biol.,* 1996, vol. 57, 157
- **HO et al.** *Gene,* 1989, vol. 77, 61
- **HORTON et al.** *Gene,* 1989, vol. 77, 61
- **SARKAR ; SOMMER.** *BioTechniques,* 1990, vol. 8, 404
- **IGLESIAS ; TRAUTNER.** *Molecular General Genetics,* 1983, vol. 189, 73-76
- **PARISH ; STOKER.** *FEMS Microbiol. Lett.,* 1997, vol. 154, 151-157
- **HOPWOOD.** The Isolation of Mutants in Methods in Microbiology. Academic Press, 1970, 363-433
- **INNIS et al.** PCR Protocols: A Guide to Methods and Application. Academic Press, 1990
- **EGON et al.** *Gene,* 1988, vol. 69, 301-315

- **VILLA-KAMAROFF et al.** *Proc. Natl. Acad. Sci. U.S.A.,* 1978, vol. 75, 3727-3731
- **DEBOER et al.** *Proc. Natl. Acad. Sci. U.S.A.,* 1983, vol. 80, 21-25
- **GILBERT et al.** *Scientific American,* 1980, vol. 242, 74-94
- **J. SAMBROOK ; E.F. FRITSCH ; T. MANIATUS.** Molecular Cloning, A Laboratory Manual. Cold Spring Harbor, 1989
- Enzyme Handbook. Springer-Verlag, 1990
- Protein Purification. VCH Publishers, 1989
- **PITCHER.** *Lett. Appl. Microbiol.,* 1989, vol. 8, 151-156
- **GILLESPIE et al.** *BMC Research Notes,* 2010, vol. 3 (81
- **IRIZARRY et al.** *Biostatistics,* 2003, vol. 4, 249-264
- Limma: Linear models for microarray data. **SMYTH.** Bioinformatics and Computational Biology Solutions using R and Bioconductor. Springer, 2005, 397-420

SEQUENCE LISTING

<110> Novozymes, Inc.
Berka, Randy
Cherry, Barbara

<120> Bacterial Mutants With Improved Transformation Efficiency

<130> 12485-WO-PCT

<150> US 61/706,6612012-09-27

<160> 18

<170> PatentIn version 3.5

<210> 1
<211> 639
<212> DNA
<213> Bacillus licheniformis

<400> 1

```
atggaaatcg aaagaataaa cgaacacacg gttaagtttt atatttccta cggtgatatc       60
gaagaccgcg ggtttgacag agaagaaatt tggtacaatc gagaacgcag tgaagagctt      120
ttttgggaaa tgatggacga agtgcacgaa gaagaagagt ttgccgttga aggccccctt      180
tggattcaag tgcaggccct tgacaaggga ttggaaatca ttgtgacaag agctcagttg      240
tccaaagacg gacaaaagct ggaactgccg attcctgaag ataaaaaaca gcatgtagca      300
gaagaaagcc ttgatgcttt gcttgacgac tttcaaaaag aagagcaggc agaagagcag      360
aaactgcagt ttgttttaaa atttgacgat tttgaggatt taatctcact gtcaaaaatg      420
tctgtcagcg gttgccaaac gacattgtac tctcatgaaa accgctatta tttattcgtg      480
gatttcaatg aactgcctga tgaagaggtg gaaaaccagc tgagcattct gctggaatat      540
gcctcagaat caaagatgac gattcatatg ctgaaagagt atggaaaact gattgcagcg      600
gatcatgctc ttcatacaat aaaaaagcac tttgcataa                              639
```

<210> 2
<211> 212
<212> PRT
<213> Bacillus licheniformis

<400> 2

```
Met Glu Ile Glu Arg Ile Asn Glu His Thr Val Lys Phe Tyr Ile Ser
1               5                   10                  15

Tyr Gly Asp Ile Glu Asp Arg Gly Phe Asp Arg Glu Glu Ile Trp Tyr
            20                  25                  30

Asn Arg Glu Arg Ser Glu Glu Leu Phe Trp Glu Met Met Asp Glu Val
        35                  40                  45
```

His Glu Glu Glu Glu Phe Ala Val Glu Gly Pro Leu Trp Ile Gln Val
    50                  55                  60

Gln Ala Leu Asp Lys Gly Leu Glu Ile Ile Val Thr Arg Ala Gln Leu
65                  70                  75                  80

Ser Lys Asp Gly Gln Lys Leu Glu Leu Pro Ile Pro Glu Asp Lys Lys
                85                  90                  95

Gln His Val Ala Glu Glu Ser Leu Asp Ala Leu Leu Asp Asp Phe Gln
            100                 105                 110

Lys Glu Glu Gln Ala Glu Glu Gln Lys Leu Gln Phe Val Leu Lys Phe
        115                 120                 125

Asp Asp Phe Glu Asp Leu Ile Ser Leu Ser Lys Met Ser Val Ser Gly
    130                 135                 140

Cys Gln Thr Thr Leu Tyr Ser His Glu Asn Arg Tyr Tyr Leu Phe Val
145                 150                 155                 160

Asp Phe Asn Glu Leu Pro Asp Glu Glu Val Glu Asn Gln Leu Ser Ile
                165                 170                 175

Leu Leu Glu Tyr Ala Ser Glu Ser Lys Met Thr Ile His Met Leu Lys
            180                 185                 190

Glu Tyr Gly Lys Leu Ile Ala Ala Asp His Ala Leu His Thr Ile Lys
        195                 200                 205

Lys His Phe Ala
    210

<210> 3
<211> 177
<212> DNA
<213> Bacillus licheniformis

<400> 3

atgaataaag ataaaaatga gaaagaagaa ttggatgagg agtggacaga cttgattaaa 60

cacgctcttg aacaaggcat tagtccagag gaaatacgta tttttctcaa tttgggaaag 120

aagtcttcaa atccttccac atcaattgaa agaagtcatt caataaatcc tttctga 177

<210> 4
<211> 58
<212> PRT
<213> Bacillus licheniformis

<400> 4

```
Met Asn Lys Asp Lys Asn Glu Lys Glu Glu Leu Asp Glu Glu Trp Thr
1               5                   10                  15

Asp Leu Ile Lys His Ala Leu Glu Gln Gly Ile Ser Pro Glu Glu Ile
            20                  25                  30

Arg Ile Phe Leu Asn Leu Gly Lys Lys Ser Ser Asn Pro Ser Thr Ser
        35                  40                  45

Ile Glu Arg Ser His Ser Ile Asn Pro Phe
    50                  55
```

<210> 5
<211> 21
<212> DNA
<213> Bacillus licheniformis

<400> 5
gaattccatt aatagctgct g 21

<210> 6
<211> 36
<212> DNA
<213> Bacillus licheniformis

<400> 6
tccatactct ttcagcatgg tcttcgatat caccgt 36

<210> 7
<211> 36
<212> DNA
<213> Bacillus licheniformis

<400> 7
acggtgatat cgaagaccat gctgaaagag tatgga 36

<210> 8
<211> 23
<212> DNA
<213> Bacillus licheniformis

<400> 8
ctcgagcgca tcctcccaaa atc 23

<210> 9
<211> 46
<212> DNA
<213> Bacillus licheniformis

<400> 9
gatagcttgg agtttctaga gcggccgcat tattaatctg ttcagc 46

<210> 10

<211> 42
<212> DNA
<213> Bacillus licheniformis

<400> 10
gagctccacc gcggtggcgg ccgctgcctt ttagtccagc tg 42

<210> 11
<211> 22
<212> DNA
<213> Bacillus licheniformis

<400> 11
gaattcgcca gaagcgaacc gc 22

<210> 12
<211> 50
<212> DNA
<213> Bacillus licheniformis

<400> 12
ccttcctttc gatattatag cacatacatt tcccccccaa aatacttgat 50

<210> 13
<211> 50
<212> DNA
<213> Bacillus licheniformis

<400> 13
atcaagtatt ttggggggga aatgtatgtg ctataatatc gaaaggaagg 50

<210> 14
<211> 21
<212> DNA
<213> Bacillus licheniformis

<400> 14
ctcgagaaat gccaaaggga g 21

<210> 15
<211> 657
<212> DNA
<213> Bacillus subtilis

<400> 15

atggaaattg aaagaattaa cgagcataca gtaaaatttt atatgtctta cggagatatt 60

gaagatcgcg gttttgacag agaagaaatt tggtataacc gtgagcgcag tgaagaactt 120

ttctgggaag tcatggatga agttcatgaa gaagaggaat tcgctgtgga aggcccactt 180

tggattcaag ttcaggcact ggacaaagga ttagaaatca tcgtcacaaa agcccagctt 240

tccaaagacg gacaaaagct cgaactgccg attcctgagg ataaaaagca agaaccagca 300

tctgaggatc ttgacgcctt gctggatgat ttccagaaag aagagcaagc cgtcaatcag 360

```
gaagagaagg agcaaaagct tcaatttgtc ttgcgatttg gcgattttga ggatgttatt      420

tctctatcta aattgaacgt taacggaagc aaaacgactt tatattcgtt tgagaaccga      480

tattatttat atgtagattt ttgcaatatg actgatgaag aggttgaaaa tcagctaagc      540

atcctgctgg agtacgcaac tgaatcctca atcagcatac accgtcttga agagtacggc      600

aagctgatta tttcagagca tgctctagaa acgataaaaa aacactttgc atcatag         657
```

<210> 16
<211> 218
<212> PRT
<213> Bacillus subtilis

<400> 16

```
Met Glu Ile Glu Arg Ile Asn Glu His Thr Val Lys Phe Tyr Met Ser
1               5                   10                  15

Tyr Gly Asp Ile Glu Asp Arg Gly Phe Asp Arg Glu Glu Ile Trp Tyr
            20                  25                  30

Asn Arg Glu Arg Ser Glu Glu Leu Phe Trp Glu Val Met Asp Glu Val
        35                  40                  45

His Glu Glu Glu Glu Phe Ala Val Glu Gly Pro Leu Trp Ile Gln Val
    50                  55                  60

Gln Ala Leu Asp Lys Gly Leu Glu Ile Ile Val Thr Lys Ala Gln Leu
65                  70                  75                  80

Ser Lys Asp Gly Gln Lys Leu Glu Leu Pro Ile Pro Glu Asp Lys Lys
                85                  90                  95

Gln Glu Pro Ala Ser Glu Asp Leu Asp Ala Leu Leu Asp Asp Phe Gln
            100                 105                 110

Lys Glu Glu Gln Ala Val Asn Gln Glu Glu Lys Glu Gln Lys Leu Gln
        115                 120                 125

Phe Val Leu Arg Phe Gly Asp Phe Glu Asp Val Ile Ser Leu Ser Lys
    130                 135                 140

Leu Asn Val Asn Gly Ser Lys Thr Thr Leu Tyr Ser Phe Glu Asn Arg
145                 150                 155                 160

Tyr Tyr Leu Tyr Val Asp Phe Cys Asn Met Thr Asp Glu Glu Val Glu
                165                 170                 175
```

```
Asn Gln Leu Ser Ile Leu Leu Glu Tyr Ala Thr Glu Ser Ser Ile Ser
            180                 185                 190

Ile His Arg Leu Glu Glu Tyr Gly Lys Leu Ile Ile Ser Glu His Ala
        195                 200                 205

Leu Glu Thr Ile Lys Lys His Phe Ala Ser
    210                 215
```

<210> 17
<211> 174
<212> DNA
<213> Bacillus subtilis

<400> 17

```
atgaagaatg caaaacaaga gcactttgaa ttggatcaag aatgggttga attaatggtg      60

gaagccaaag aggcaaatat cagcccggaa gaaatacgaa aatatttact tttaaacaaa     120

aagtctgctc atcctggtcc ggcagccaga agtcataccg taaatccttt ctga           174
```

<210> 18
<211> 57
<212> PRT
<213> Bacillus subtilis

<400> 18

```
Met Lys Asn Ala Lys Gln Glu His Phe Glu Leu Asp Gln Glu Trp Val
1               5                   10                  15

Glu Leu Met Val Glu Ala Lys Glu Ala Asn Ile Ser Pro Glu Glu Ile
            20                  25                  30

Arg Lys Tyr Leu Leu Leu Asn Lys Lys Ser Ala His Pro Gly Pro Ala
        35                  40                  45

Ala Arg Ser His Thr Val Asn Pro Phe
    50                  55
```